# EUROPEAN PATENT APPLICATION

(11) **EP 3 876 184 A1**
(43) Date of publication of application: **08.09.2021**
(21) Application number: 19878680.8
(22) Date of filing: 01.11.2019
(51) Int. Cl.: G06Q 50/22, G16H 50/30

(54) **METHOD, SYSTEM, AND PROGRAM FOR CREATING HEALTH POSITIONING MAP AND HEALTH FUNCTION, AND METHOD FOR USING THESE**

(30) Priority: 02.11.2018 JP 2018207611
(71) Applicant: RIKEN, Wako-shi Saitama 351-0198 (JP)
(72) Inventor: WATANABE Yasuyoshi, Wako-shi, Saitama 351-0198 (JP); MIZUNO Kei, Wako-shi, Saitama 351-0198 (JP); KUME Satoshi, Wako-shi, Saitama 351-0198 (JP); WATANABE Kyosuke, Wako-shi, Saitama 351-0198 (JP); TANEISHI Kei, Wako-shi, Saitama 351-0198 (JP)
(74) Representative: Uexküll & Stolberg
(86) International application number: PCT/JP2019/043062
(87) International publication number: WO 2020/091053

(57) **Abstract**

The present invention provides a method for creating a health positioning map, the method including: acquiring a first data set for a first parameter set, for each of a plurality of examinees; processing the first data set to obtain first data; mapping the processed first data for each of the plurality of examinees; clustering the mapped first data and thereby specifying a plurality of regions; and characterizing at least some of the plurality of regions.

## Description

### [Technical Field]

The present invention relates to a method and the like of creating a health positioning map and a health function.

### [Background Art]

The preliminary stage before reaching onset of a disease from a healthy state is referred to as ahead sick. In view of concerns for national economic collapse due to increased medical expenses in a super aged society with a falling birthrate, development of a technology that enables visualization/quantification of a pre-disease state from a heathy state to prevent onset of a disease in advance is desired.

For example, Patent Literature 1 and Patent Literature 2 disclose a technology for evaluating the health status of a subject by acquiring data for a plurality of test items from a plurality of subjects and creating a function using a plurality of pieces of test data for the test items as variables.

### [Citation List]

### [Patent Literature]

[PTL 1] Japanese Laid-Open Publication No. 2010-230428
[PTL 2] Japanese Patent No. 6069598
[PTL 3] Japanese Patent No. 5455071
[PTL 4] Japanese Patent No. 5491749

### [Summary of Invention]

### [Technical Problem]

However, the aforementioned conventional art evaluates risk by taking measurements that follow a path of heathy state, pre-disease state, and disease along a specific axis such as lifestyle diseases including diabetes and arteriosclerosis, cancer, dementia, sarcopenia, liver disease, or renal disease. For this reason, the conventional art has problems such as the inability to evaluate the degree of overall health of an individual.

The objective of one embodiment of the invention is to provide means of evaluating various health risks (e.g., health function creation method, health function creation apparatus, or the like that can create health function) to express the overall health level of an individual.

### [Solution to Problem]

To solve the problem described above, a health function creation method according to one embodiment of the invention is a health function creation method for creating a health function of a group of subjects, comprising a first data acquisition step for acquiring first data related to health comprising future health diagnosis item data for the group of subjects, and a health function creation step for creating a heath function using the first data.

To solve the problem described above, a health function creation apparatus according to one embodiment of the invention is a health function creation apparatus for creating a health function of a group of subjects, comprising a data acquisition unit for acquiring first data related to health comprising future health diagnosis item data for the group of subjects, and a health function creation unit for creating a heath function using the first data.

The present invention provides, for example, the following items.
(Item 1)
   A method of creating a health positioning map, comprising:
   acquiring a first data set with respect to a first parameter set for each of a plurality of subjects;
   processing the first data set to obtain first data;
   mapping the processed first data for each of the plurality of subjects;
   clustering the mapped first data to identify a plurality of regions; and
   characterizing at least some of the plurality of regions.
(Item 2)
   The method of item 1, wherein the first parameter set comprises an autonomic nerve parameter, a biological oxidation parameter, a less biological repair energy parameter, and an inflammation parameter.
(Item 3)
   The method of item 2, wherein the first parameter set further comprises a fundamental parameter, a cognitive function parameter, and a subjective parameter.
(Item 4)
   The method of any one of items 1 to 3, wherein the processing of the first data set comprises dimensionality reduction processing of the first data set.
(Item 5)
   The method of any one of items 1 to 4, wherein the processing of the first data set comprises standardization of the first data set and dimensionality reduction processing of the standardized data set.
(Item 6)
   The method of item 5, wherein the standardization of the first data set comprises:
   classifying the first data set into a data set for a male subject and a data set for a female subject; and
   standardizing the dataset for a male subject and/or standardizing the data set for a female subject.
(Item 7)
   The method of any one of items 4 to 6, wherein the dimensionality reduction processing is performed by multidimensional scaling.
(Item 8)
   The method of any one of items 1 to 7, further comprising:
   selecting the regions;
   designating the first data mapped to the regions as sub-first data;
   clustering the sub-first data to identify a plurality of regions; and
   characterizing at least some of the plurality of regions.
(Item 9)
   The method of any one of items 1 to 8, wherein the health positioning map is a two-dimensional or three-dimensional map.
(Item 10)
   A method of creating a health function for mapping a health level of a subject onto a health positioning map, comprising:
   preparing a health positioning map created by the method of any one of items 1 to 9;
   acquiring a second data set with respect to a second parameter set for at least some of the plurality of subjects, the second parameter set being a part of the first parameter set; and
   deriving a health function that correlates the second data set with a position on the health positioning map of the at least some of the subjects.
(Item 11)
   The method of item 10, wherein the derivation is performed by machine learning.
(Item 12)
   The method of item 10 or 11, wherein the second parameter set does not comprise a result of an invasive test.
(Item 13)
   The method of item 12, wherein the second parameter set comprises:
   (1) age;
   (2) BMI;
   (3) fat percentage;
   (4) SOS;
   (5) systolic blood pressure;
   (6) subjective evaluation on fatigue;
   (7) subjective evaluation on depression;
   (8) activity of a parasympathetic nerve;
   (9) activity of an entire autonomic nervous system; and
   (10) cognitive function.
(Item 14)
   The method of item 10 or 11, wherein the second parameter set comprises age, subjective evaluation on fatigue, fatigue duration, balance between a sympathetic nerve and a parasympathetic nerve, cognitive function, fat percentage, blood neutral fat, blood oxidative stress index (OSI), and blood CRP.
(Item 15)
   The method of any one of items 10 to 14, wherein the health function is a linear regression model or a nonlinear regression model using the second data set as an independent variable and a position on the health positioning map of the at least some of the subjects as a dependent variable.
(Item 16)
   A method of estimating a health level of a user, comprising:
   preparing a health function created by the method of any one of items 10 to 15;
   acquiring a first user data set with respect to the second parameter set of the user;
   obtaining a first output by inputting the first user data set into the health function; and
   mapping the first output onto the health positioning map.
(Item 17)
   The method of item 16, comprising:
   acquiring a second user data set with respect to the second parameter set of the user after a predetermined period has elapsed;
   obtaining a second output by inputting the second user data set into the health function; and
   mapping the second output onto the health positioning map.
(Item 18)
   A method of evaluating an item for improving a health status, comprising:
   comparing the first output with the second output in the method of item 17;
   wherein the user uses the item during the predetermined period.
(Item 19)
   A method of creating a health function for mapping a health level of a subject on a health positioning map, comprising:
   preparing a health positioning map created by using a first parameter set;
   acquiring a second data set with respect to a second parameter set, the second parameter set being a part of the first parameter set; and
   deriving a health function that correlates the second data set with a position on the health positioning map of a subject.
(Item 20)
   The method of item 19, wherein the first parameter set comprises an autonomic nerve parameter, a biological oxidation parameter, a less biological repair energy parameter, and an inflammation parameter.
(Item 21)
   The method of item 19 or 20, wherein the derivation is performed by machine learning.
(Item 22)
   The method of any one of items 19 to 21, wherein the second parameter set does not comprise a result of an invasive test.
(Item 23)
   The method of item 22, wherein the second parameter set comprises:
   (1) age;
   (2) BMI;
   (3) fat percentage;
   (4) SOS;
   (5) systolic blood pressure;
   (6) subjective evaluation on fatigue;
   (7) subjective evaluation on depression;
   (8) activity of a parasympathetic nerve;
   (9) activity of an entire autonomic nervous system; and
   (10) cognitive function.
(Item 24)
   The method of any one of items 19 to 21, wherein the second parameter set comprises age, subjective evaluation on fatigue, fatigue duration, balance between a sympathetic nerve and a parasympathetic nerve, cognitive function, fat percentage, blood neutral fat, blood oxidative stress index (OSI), and blood CRP.
(Item 25)
   A method of estimating a health level of a user, comprising:
   acquiring a first user data set with respect to a second parameter set of the user;
   obtaining a first output by inputting the first user data set into a health function; and
   mapping the first output onto a health positioning map;
   wherein the health function is a function that correlates the user data set with a position on the health positioning map.
(Item 26)
   The method of item 25, wherein the second parameter set does not comprise a result of an invasive test.
(Item 27)
   The method of item 26, wherein the second parameter set comprises:
   (1) age;
   (2) BMI;
   (3) fat percentage;
   (4) SOS;
   (5) systolic blood pressure;
   (6) subjective evaluation on fatigue;
   (7) subjective evaluation on depression;
   (8) activity of a parasympathetic nerve;
   (9) activity of an entire autonomic nervous system; and
   (10) cognitive function.
(Item 28)
   The method of item 25, wherein the second parameter set comprises age, subjective evaluation on fatigue, fatigue duration, balance between a sympathetic nerve and a parasympathetic nerve, cognitive function, fat percentage, blood neutral fat, blood oxidative stress index (OSI), and blood CRP.
(Item 29)
   The method of any one of items 25 to 28, wherein the health positioning map is created using a first parameter set, and the first parameter set comprises an autonomic nerve parameter, a biological oxidation parameter, a less biological repair energy parameter, and an inflammation parameter.
(Item 30)
   The method of item 29, wherein the first parameter set further comprises a fundamental parameter, a cognitive function parameter, and a subjective parameter.
(Item 31)
   A system for creating a health positioning map, comprising:
   acquisition means for acquiring a first data set with respect to a first parameter set for each of a plurality of subjects;
   processing means for processing the first data set to obtain first data;
   mapping means for mapping the processed first data for each of the plurality of subjects;
   clustering means for clustering the mapped first data to identify a plurality of regions; and
   characterization means for characterizing at least some of the plurality of regions.
(Item 32)
   A system for creating a health function for mapping a health level of a subject on a health positioning map, comprising:
   first acquisition means for acquiring a health positioning map created by the system of item 31;
   second acquisition means for acquiring a second data set with respect to a second parameter set for at least some of the plurality of subjects, the second parameter set being a part of the first parameter set; and
   derivation means for deriving a health function that correlates the second data set with a position on the health positioning map of the at least some of the subjects.
(Item 33)
   A system for estimating a health level of a user, comprising:
   third acquisition means for acquiring a health function created by the system of item 32;
   fourth acquisition means for acquiring a first user data set with respect to the second parameter set of the user;
   output generation means for generating a first output by inputting the first user data set into the health function; and
   output mapping means for mapping the first output onto the health positioning map.
(Item 34)
   A system for creating a health function for mapping a health level of a subject onto a health positioning map, comprising:
   first acquisition means for acquiring a health positioning map created by using a first parameter set;
   second acquisition means for acquiring a second data set with respect to a second parameter set, the second parameter set being a part of the first parameter set; and
   derivation means for deriving a health function that correlates the second data set with a position on the health positioning map of a subject.
(Item 35)
   A system for estimating a health level of a user, comprising:
   acquisition means for acquiring a first user data set with respect to a second parameter set of the user;
   output generation means for generating a first output by inputting the first user data set into a health function; and
   output mapping means for mapping the first output onto a health positioning map;
   wherein the health function is a function that correlates the user data set with a position on the health positioning map.
(Item 36)
   A program for creating a health positioning map, the program being executed in a computer system comprising a processing unit, the program causing the processing unit to perform processing comprising:
   acquiring a first data set with respect to a first parameter set for each of a plurality of subjects;
   processing the first data set to obtain first data;
   mapping the processed first data for each of the plurality of subjects;
   clustering the mapped first data to identify a plurality of regions; and
   characterizing at least some of the plurality of regions.
(Item 37)
   A program for creating a health function for mapping a health level of a subject on a health positioning map, the program being executed in a computer system comprising a processing unit, the program causing the processing unit to perform processing comprising:
   acquiring a health positioning map created by executing the program of item 36;
   acquiring a second data set with respect to a second parameter set for at least some of the plurality of subjects, the second parameter set being a part of the first parameter set; and
   deriving a health function that correlates the second data set with a position on the health positioning map of the at least some of the subjects.
(Item 38)
   A program for estimating a health level of a user, the program being executed in a computer system comprising a processing unit, the program causing the processing unit to perform processing comprising:
   acquiring a health function created by executing the program of item 37;
   acquiring a first user data set with respect to the second parameter set of the user;
   obtaining a first output by inputting the first user data set into the health function; and
   mapping the first output onto the health positioning map.
(Item 39)
   A program for creating a health function for mapping a health level of a subject onto a health positioning map, the program being executed in a computer system comprising a processing unit, the program causing the processing unit to perform processing comprising:
   acquiring a health positioning map created by using a first parameter set;
   acquiring a second data set with respect to a second parameter set, the second parameter set being a part of the first parameter set; and
   deriving a health function that correlates the second data set with a position on the health positioning map of a subject.
(Item 40)
   A program for estimating a health level of a user, the program being executed in a computer system comprising a processing unit, the program causing the processing unit to perform processing comprising:
   acquiring a first user data set with respect to a second parameter set of the user;
   obtaining a first output by inputting the first user data set into a health function; and
   mapping the first output onto a health positioning map;
   wherein the health function is a function that correlates the user data set with a position on the health positioning map.

### [Advantageous Effects of Invention]

One embodiment of the invention can provide means capable of evaluating various health risks (e.g., health positioning map, health function, or the like).

### [Brief Description of Drawings]

[Figure 1A] Figure **1A** is a diagram showing an example of a flow of a new service for making a health status of a user viewable.
[Figure 1B] Figure **1B** is a diagram showing an example of screen **1000** showing a health status of a user.
[Figure 2] Figure **2** is a diagram showing an example of a configuration of computer system **100.**
[Figure 3A] Figure **3A** is a diagram showing an example of a configuration of processing unit **120** in one embodiment.
[Figure 3B] Figure **3B** is a diagram showing an example of a configuration of processing unit **130** in another embodiment.
[Figure 3C] Figure **3C** is a diagram showing an example of a configuration of processing unit **140** in still another embodiment.
[Figure 3D] Figure **3D** is a diagram showing an example of a data flow in one embodiment of the invention.
[Figure 4] Figure **4** is a diagram showing an example of a data configuration of a first data set stored in database unit **200.**
[Figure 5A] Figure **5A** is a flowchart showing an example of processing in the computer system **100.**
[Figure 5B] Figure **5B** is a flowchart showing another example of processing in the computer system **100.**
[Figure 6] Figure **6** is a flowchart showing another example of processing in the computer system **100.**
[Figure 7A] Figure **7A** is a flowchart showing another example of processing in the computer system **100.**
[Figure 7B] Figure **7B** is a flowchart showing an example of processing following processing **700** shown in Figure **7A****.**
[Figure 8] Figure **8** is a diagram showing an example of a health evaluation map created by the health evaluation apparatus described above.
[Figure 9] Figure **9** is a diagram showing a result of clustering a plot distribution pattern in this Example.
[Figure 10] Figure **10** is a diagram showing a health positioning map created in one Example.
[Figure 11] Figure **11** is a diagram showing a correlation coefficient between an actual measurement value and a predicted value of each of a health function using a second parameter set of 50 items, health function using a second parameter set of 21 items, and health function using a second parameter set of 9 items.
[Figure 12] Figure **12** is a diagram showing a correlation coefficient between an actual measurement value and a predicted value of a health function using a second parameter set of 15 noninvasive items.
[Figure 13] Figure **13** is a diagram showing a result of comparing mapping positions on a health positioning map before dosing of reduced Coenzyme Q10 (CoQ₁₀) and after dosing thereof for three months.
[Figure 14] Figure **14** is a diagram showing a result comparing parameters of test items before dosing of reduced CoQ₁₀ and after dosing thereof for three months.

### [Description of Embodiments]

The embodiments of the invention are described hereinafter with reference to the drawings. As used herein, "about" refers to a range of ± 10% from the numerical value that is described subsequent to "about".

### 1. Service for making the health status of user viewable

The inventors of the present invention developed a new service for making a health status of a user viewable. This is a service, which acquires various pieces of data related to the health of a user, and provides the user with information regarding which region on a health positioning map the health level of the user is positioned, based on the acquired data. The health positioning map herein refers to a map with a plurality of regions characterized with information related to health.

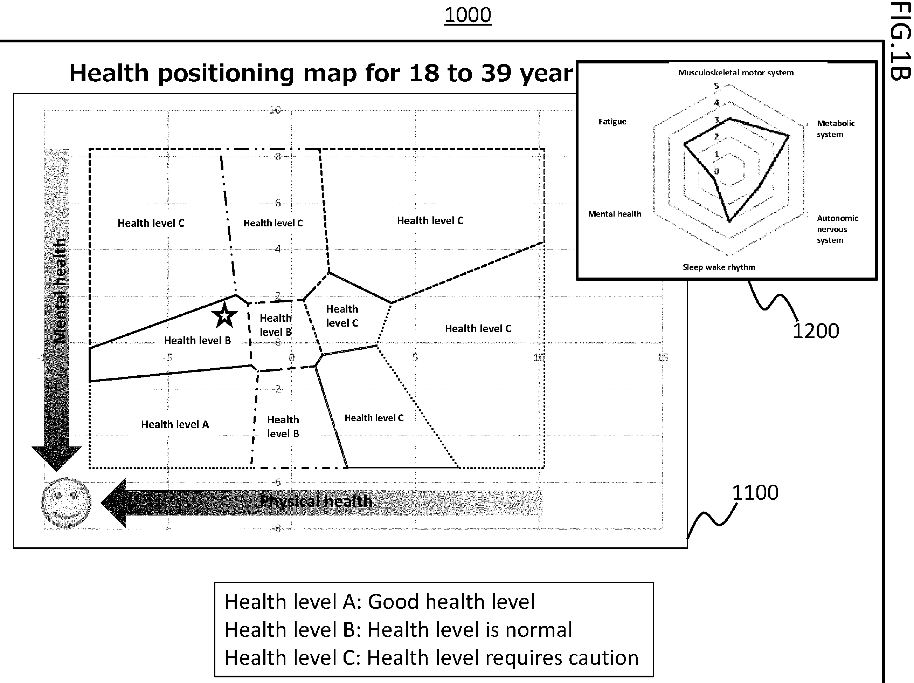

Each region on a health positioning map can be characterized by, for example, various states of health levels. For example, a region on a health positioning map can be characterized as a young mental health disease risk group, and another region on the health positioning map can be characterized as a middle age-senior life style disease risk group. A still another region on the health positioning map can be characterized as a senior diabetes risk group. For example, a region on a health positioning map can be characterized as a pre-disease state group for a certain health status, and another region can be characterized as a high risk group for the health status. Such characterizations are examples. Various characterizations can be applied. For example, as shown in Figure **1B** described below, regions can be characterized as health levels A to C with physical health and mental health as axes. Such characterization is simple, and the status of health level can be intuitively understood. Characterization of each region on a health positioning map can be determined by analyzing a trend of a plurality of subjects belonging to each region.

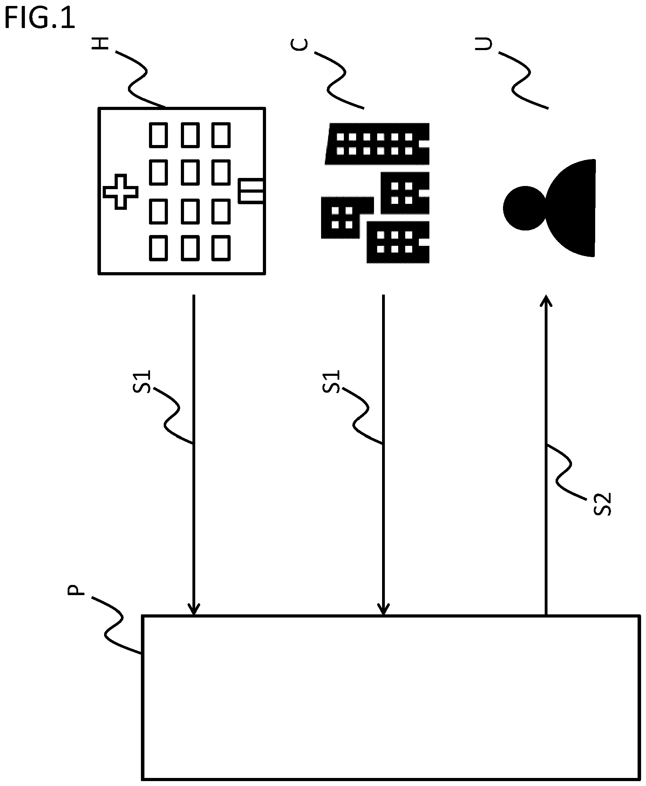

Figure **1A** shows an example of a flow of a new service for making a health status of a user viewable.

At step **S1**, user **U** undergoes testing at facility **H** such as a hospital or a specialized testing facility, and the result of the test is provided to a service provider **P.** The test at the facility **H** can include a noninvasive test such as medical consultation or cognitive function test in addition to an invasive test such as a blood test. Alternatively at step **S1**, the user **U** undergoes a simple test at facility **C** such as a company that is not a testing facility, and the result of the test is provided to the service provider **P.** The simple test at facility **C** can include, for example, only noninvasive tests such as medical consultation and a cognitive function test. At step **S1**, it is not necessary to administer both an invasive test and a noninvasive test administered at facility **H** and a noninvasive test administered at facility **C.** Only a test at facility **H** or only a simple test at facility **C** may be administered. As used herein, "invasive test" refers to a test that damages the body of a test subject (e.g., by blood collection using a syringe or tissue resection), and "noninvasive test" refers to a test involving no damage to the body of a test subject. A typical invasive test is a test for detecting the amount of components contained in blood or plasma. A typical noninvasive test is a test for detecting a component in a discharge (urine, breath, or saliva) of a subject, autonomic nervous function test, cognitive function test, questionnaire/VAS (Visual Analogue Scale), or the like. As used herein, "invasive parameter" refers to a parameter obtained through an invasive test, and "noninvasive parameter" refers to a parameter obtained through a noninvasive test.

The service provider **P** can generate information regarding which region on a health positioning map the health level of the user is positioned, based on the provided test result of the user.

At step **S2,** information generated by the service provider **P** is provided to the user. The user can be aware of the user's own health status by referring to the information associated with a region on a health positioning map in which the user's own health level is positioned. For example, if the user's health level belongs to a region characterized as health level B on a health positioning map as shown in Figure **1B****,** the user can be aware that the user's own health level is not bad but not good. For example, this can urge the user to try to improve the user's own health level. If, for example, the user's health level belongs to a region characterized as a young mental health disease risk group on a health positioning map, the user can be aware of the user's own health status as being at risk for a mental health disease. For example, this can urge the user to take measures to address the risk of a mental health disease.

The user can further be aware of a chronological change in the user's own health status by repeating the aforementioned step **S1** to step **S2** after a predetermined period has elapsed. For example, the user can be aware of the direction toward which the user's own health status is headed by referring to chronological changes in the position on a health positioning map. If, for example, the user's health level belonged to a region characterized as health level B on a health positioning map as shown in Figure **1B****,** but after a predetermined period has elapsed, the user's health level, although still belonging to a region characterized as health level B on the health positioning map, has moved closer to a region characterized as health level C, the user can be aware that the user's own health status is heading toward the direction of health level C. For example, this can urge the user to try to prevent the user's health level from deteriorating. If, for example, the user's health level belonged to a region characterized as a young mental health disease risk group on a health positioning map, but after a predetermined period has elapsed, the user's health level, although still belonging to a region characterized as a young mental health disease risk group on a health positioning map, has moved closer to a region characterized as a middle age to senior life style disease risk group, the user can be aware that the user's own health status is heading toward the direction of risk for a middle age to senior life style disease. For example, this can urge the user to take measures to address the risk for a life style disease.

Figure **1B** shows an example of screen **1000** showing a health status of a user. The screen **1000** can be displayed and provided to a user, for example, on a display screen of a terminal apparatus of the user (e.g., personal computer, smart phone, tablet, or the like).

The screen **1000** comprises a health positioning map display section **1100** and a radar chart display section **1200.**

The health positioning map display section **1100** displays a health positioning map. On the health positioning map, the horizontal axis is associated with physical health, where a greater value on the horizontal axis indicates worse physical health, while the vertical axis is associated with mental health, where a greater value on the vertical axis indicates worse mental health.

A health positioning map displayed on the health positioning map display section **1100** includes 10 regions. Among the 10 regions, 1 region is characterized as health level A, 3 regions are characterized as health level B, and 6 regions are characterized as health level C. In this regard, health level A indicates a good health level, health level B indicates that the health level is normal, and health level C indicates a poor health level requiring caution.

The user can be aware of the user's own health status in accordance with which region on a health positioning map the user's health level is positioned. In the example shown in Figure **1B****,** the use's health level is plotted with a star symbol. It can be understood that the user's health level belongs to a region characterized as health level B.

The radar chart display section **1200** displays a radar chart. The radar chart shows the health status in 6 levels from 0 to 5 from 6 viewpoints (musculoskeletal motor system, metabolic system, autonomic nervous system, sleep wake rhythm, mental health, and fatigue). The viewpoint of musculoskeletal motor system indicates the status of muscle or the like associated with the motor function. The viewpoint of metabolic system indicates the status of energy metabolism in the body, obesity, or the like. The viewpoint of autonomic nervous system indicates the status of the ability to regulate the nerves associated with concentration or relaxation. The viewpoint of sleep wake rhythm indicates the status of sleep, drowsiness, or the like. The viewpoint of mental health indicates the status such as depressed mood. The viewpoint of fatigue indicates the status of mental or physical exhaustion. The user can be aware of which viewpoint the user's health status is attributed to at a glance. Each axis of the radar chart displayed on the radar chart display section **1200** is characterized by information related to health, and the score of the user is mapped to each axis. Therefore, such a radar chart can also be considered as a type of a health positioning map herein.

The aforementioned service can be materialized, for example, by the computer system **100** described below.

### 2. Configuration of computer system

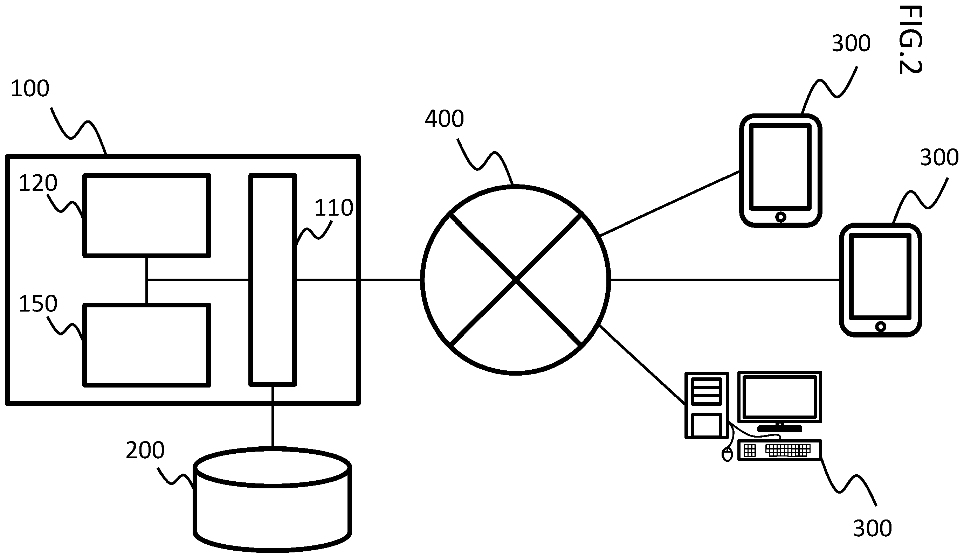

Figure **2** shows an example of a configuration of the computer system **100.**

The computer system **100** is connected to a database unit **200.** The computer system **100** is also connected to at least one user terminal apparatus **300** via a network **400.**

The network **400** can be any type of network. The network **400** can be, for example, the Internet or LAN. The network **400** can be a wired network or a wireless network.

Examples of the computer system **100** include, but are not limited to, a computer (e.g., server apparatus) installed at a service provider providing a new service for making a health status of a user viewable. Examples of the user terminal apparatus **300** include, but are not limited to, a computer installed at a hospital (e.g., terminal apparatus), a computer installed in one room of an office that can administer a test (e.g., terminal apparatus), and a computer held by a user (e.g., terminal apparatus). In this regard, the computer (server apparatus or terminal apparatus) can be any type of computer. A terminal apparatus can be, for example, any type of terminal apparatus such as a smart phone, tablet, personal computer, or a smart glass.

The computer system **100** comprises an interface unit **110,** a processing unit **120,** and a memory unit **150.**

The interface unit **110** exchanges information with the outside of the computer system **100.** The processing unit **120** of the computer system **100** can receive information from the outside of the computer system **100** via the interface unit **110** and transmit information to the outside of the computer system **100.** The interface unit **110** can exchange information in any manner.

The interface **110** comprises, for example, an input unit that enables input of information into the computer system **100.** The mode through which an input unit enables input of information into the computer system **100** is not limited. If, for example, the input unit is a touch panel, a user can input information by touching the touch panel. Alternatively, if the input unit is a mouse, a user can input information by operating the mouse. Alternatively, if the input unit is a keyboard, a user can input information by pressing a key on the keyboard. Alternatively, if the input unit is a microphone, a user can input information by inputting an audio into the microphone. Alternatively, if the input unit is a camera, the input unit can input information captured by the camera. Alternatively, if the input unit is a data reader, information can be inputted by reading out information from a storage medium connected to the computer system **100.** Alternatively, if the input unit is a receiver, information can be inputted by the receiver receiving the information from the outside of the computer system **100** via the network **400.**

The interface unit **110** comprises, for example, an output unit that enables output of information from the computer system **100.** The mode through which an output unit enables output of information from the computer system 100 is not limited. If, for example, the output unit is a display screen, information can be outputted onto the display screen. Alternatively, if the output unit is a speaker, information can be outputted by an audio from the speaker. Alternatively, if the output unit is a data writing apparatus, information can be outputted by writing in information into a storage medium connected to the computer system **100.** Alternatively, if the output unit is a transmitter, information can be outputted by the transmitter transmitting the information to the outside of the computer system **100** via the network **400.** In such a case, the type of network is not limited. For example, the transmitter can transmit information via the Internet or LAN.

The processing unit **120** executes processing of the computer system **100** and controls the overall operation of the computer system **100.** The processing unit **120** reads out a program stored in the memory unit **150** and executes the program. This can cause the computer system **100** to function as a system that executes a desired step. The processing unit **120** can be implemented by a single processor or a plurality of processors.

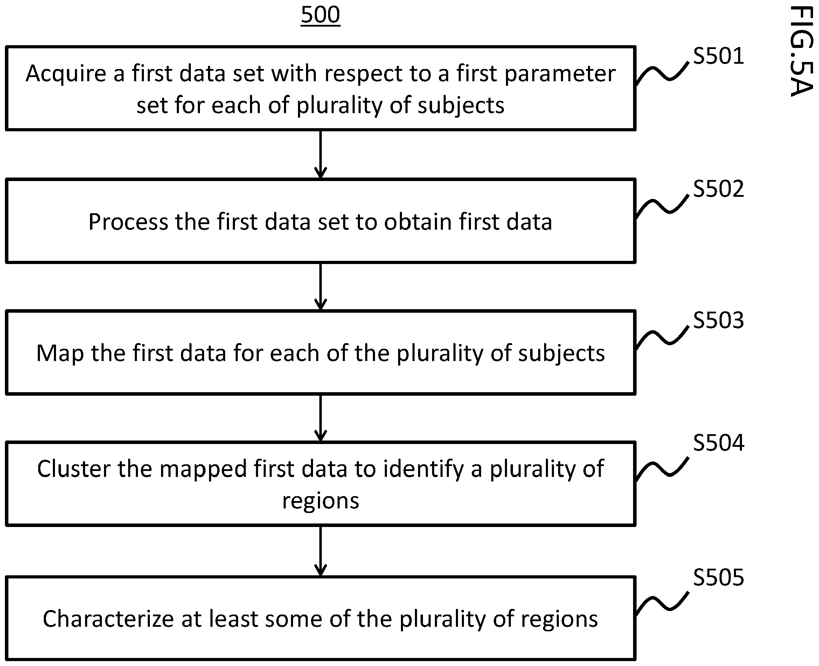

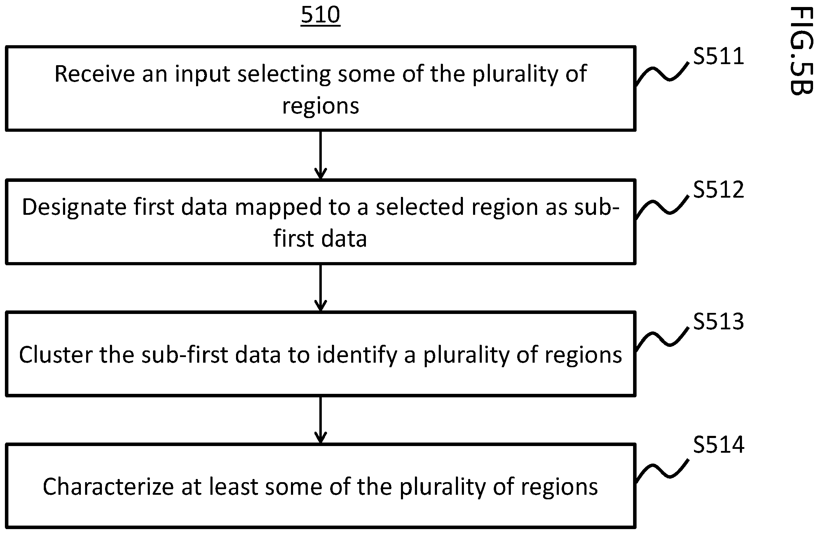

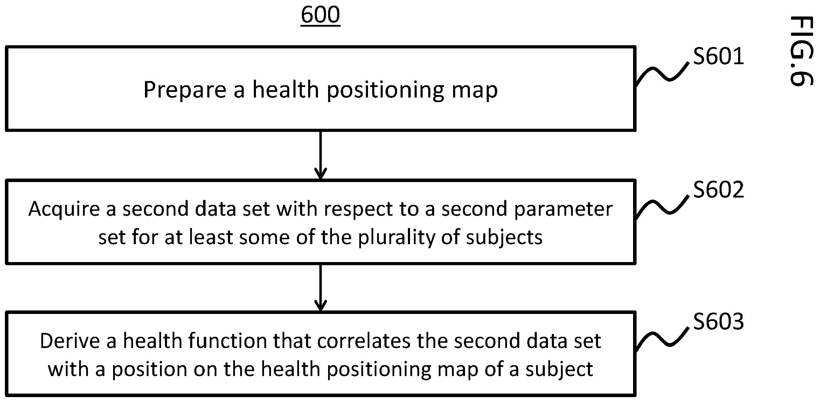

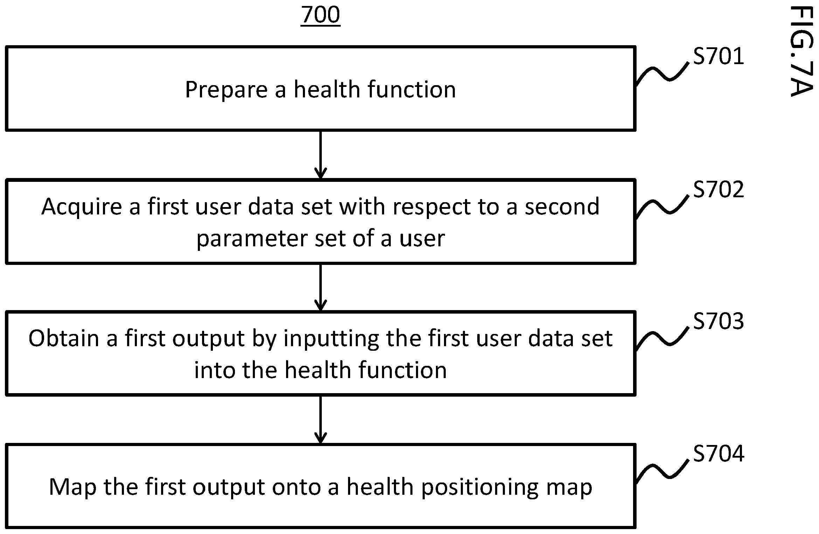

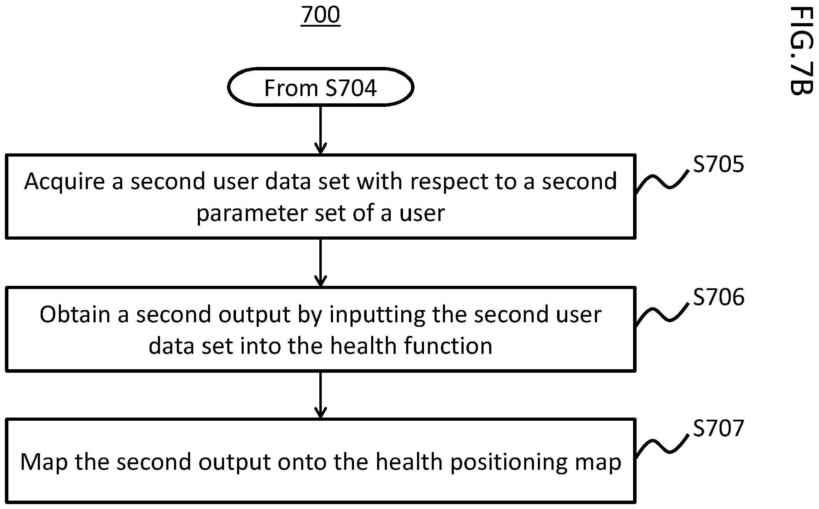

The memory unit **150** stores a program required for executing the processing of the computer system **100,** data required for executing the program, and the like. The memory unit **150** can store a program for causing the processing unit **120** to perform processing for creating a health positioning map (e.g., program materializing the processing shown in Figure **5A** and Figure **5B** described below), a program for causing a processing unit **130** to perform processing for creating a health function (e.g., program materializing the processing shown in Figure **6** described below), or a program for causing a processing unit **140** to perform processing for estimating a health level of a user (e.g., program materializing the processing shown in Figure **7A** and Figure **7B** described below). In this regard, a program can be stored in the memory **150** in any manner. For example, a program can be preinstalled in the memory **150.** Alternatively, a program can be installed in the memory unit **150** by being downloaded through a network. In such a case, the type of network is not limited. The memory unit **150** can be implemented by any storage means.

For example, data obtained from a plurality of subjects can be stored in the database unit **200.** For example, data for a health positioning map generated by the computer system **100** can be stored in the database unit **200.** For example, a health function generated by the computer system **100** can be stored in the database unit **200.**

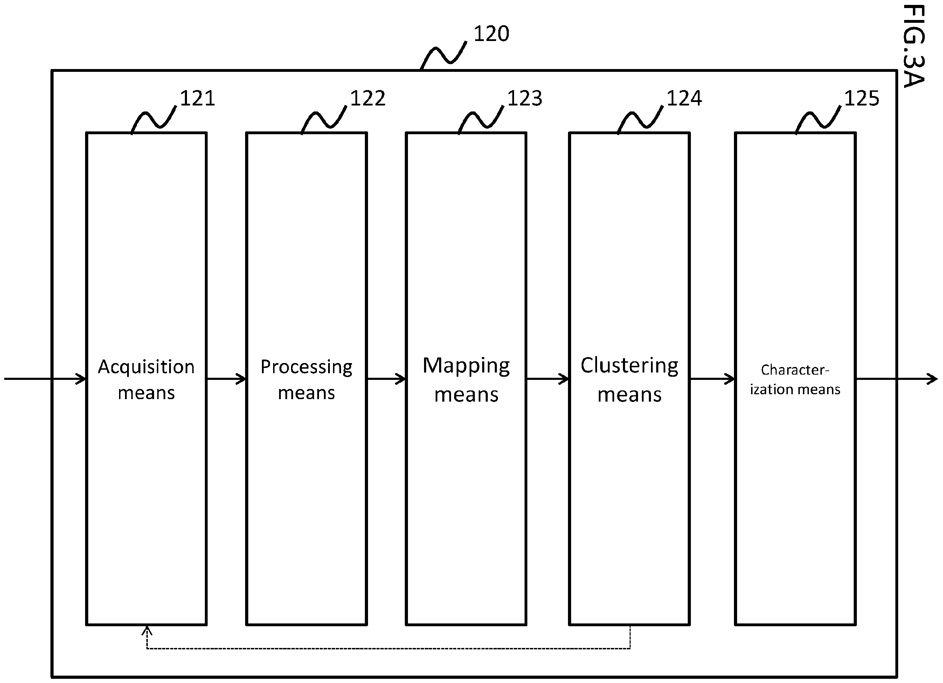

Figure **3A** shows an example of a configuration of the processing unit **120** in one embodiment. The processing unit **120** can have a configuration for processing that creates a health positioning map.

The processing unit **120** comprises acquisition means **121,** processing means **122,** mapping means **123,** clustering means 124, and characterization means **125.**

The acquisition means **121** is configured to acquire a first data set with respect to a first parameter set described below for each of a plurality of subjects. For example, the acquisition means **121** acquire a dataset with respect to a plurality of items (e.g., 232 items in a certain embodiment) for each subject. The first parameter set can be a parameter set obtained by, for example, acquiring a data set with respect to an initial parameter set, finding a correlation between each data of the data set with respect to the initial parameter set, and extracting a parameter with a correlation coefficient that is equal to or greater than a predetermined threshold value. At this time, an extracted parameter set can be extracted to include the four basic parameters described below.

The acquisition means **121** can, for example, receive data for a plurality of subjects stored in the database unit **200** via the interface unit **110** to acquire the received data. The acquisition means **121** can, for example, receive data for a plurality of subjects stored in the database unit **200** from a computer system of a test facility (e.g., hospital, research laboratory, or the like) via the interface unit **110** to acquire the received data. The acquired first data set is passed along to the processing means **122** for subsequent processing.

A first data set with respect to a first parameter set can be stored in the database unit **200.**

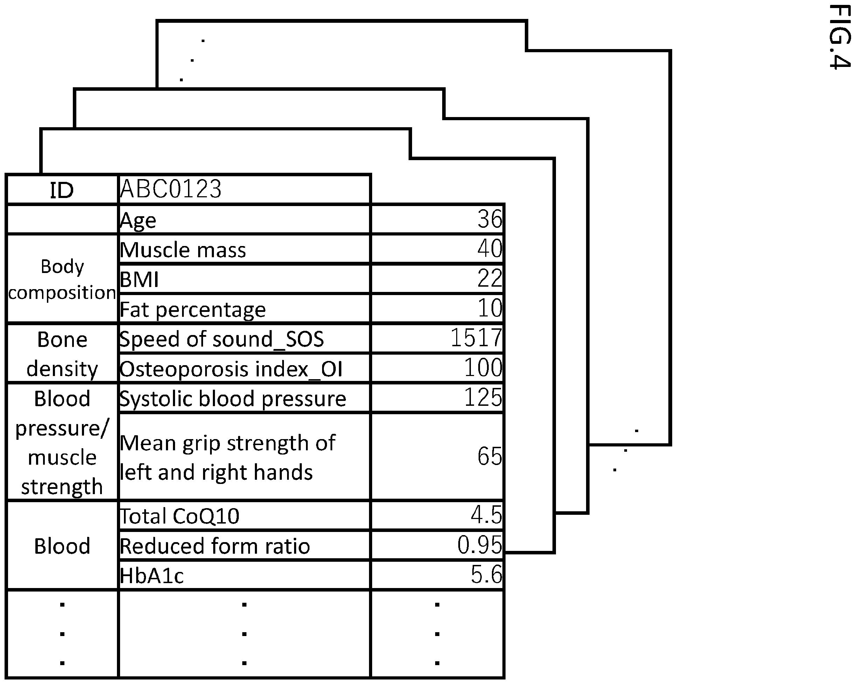

Figure **4** shows an example of a data configuration of a first data set stored in the database unit **200.**

A first data set with respect to a first parameter set for each of a plurality of subjects is stored in the database unit **200.** An ID is assigned to each of the plurality of subjects. For example, a set (first data set) of values of each parameter of the first parameter set such as age, muscle mass, BMI, fat percentage, speed of sound, osteoporosis index ... is stored in the database unit **200.**

Referring back to Figure **3A****,** the acquisition means **121,** in one embodiment, can be configured to further acquire first data contained in some of a plurality of regions contained in a created health positioning map as sub-first data. Alternatively, the acquisition means **121** can be configured to acquire first data contained in some of a plurality of regions identified by the clustering means **124** described below as sub-first data. The acquisition means **121** can, for example, acquire first data within a region selected by a health positioning map creator. A health positioning map creator can input a selection of a region into the computer system **100** via the interface unit **110.** A health positioning map creator can, for example, select a region to which a specific population among a plurality of subjects, such as a male subject population, female subject population, young population (population of subjects under 40 years old), middle age population (population of subjects who are 40 or older and younger than 60 years old), or senior population (population of subjects who are 60 or older), can belong, in order to create a health positioning map focusing on such a population. The acquired sub-first data can be passed along to the clustering means **124** for subsequent processing.

The processing means **122** is configured to process a first data set acquired by the acquisition means **121.** The processing means **122** can output first data by processing a first data set.

Processing by the processing means **122** can include any processing, as long as the outputted first data can be mapped. When creating a positioning map based on data for both males and females, it is preferable to apply a correction for the difference between sexes.

Processing by the processing means **122** can include, for example, dimensionality reduction processing. Dimensionality reduction processing is processing that converts an m-dimensional data into n-dimensional data, wherein m > n. Dimensionality reduction processing can be performed using, for example, multi-dimensional scaling (MDS), principal component analysis, multiple regression analysis, principle component analysis, machine learning, or the like, but the dimensionality reduction processing means is not limited thereto. Dimensionality reduction processing preferably reduces a first data set to two dimensional data or three dimensional data. This is because when two dimensional data or three dimensional data is mapped by the mapping means **123** described below, a map is created in a two dimensional space or a three dimensional space, so that a visually readily understandable map can be obtained. Dimensionality reduction processing can be performed using multidimensional scaling. This is because mapping first data obtained by multidimensional scaling by the mapping means **123** described below can result in a visually readily understandable map.

Processing by the processing means **122** can include, for example, standardization processing. Standardization processing is processing that aligns the scale of data for each parameter of a first data set. Standardization processing can be, for example, processing that computes a Z score (processing that corrects data so that the mean value is 0 and the standard deviation is 1), processing that computes a T score (processing that corrects data so that the mean value is 50 and the standard deviation is 10), or the like. The processing means **122** can be configured to perform standardization processing on data of a first data set with respect to all parameters in a first parameter set, or with respect to a specific parameter.

The processing means **122** can be configured to perform standardization processing on a first data set for all of the plurality of subjects, or for a specific population among the plurality of subjects. Examples of the specific population among the plurality of subjects include, but are not limited to, a male subject population, a female subject population, a young population (population of subjects under 40 years old), a middle age population (population of subjects who are 40 or older and younger than 60 years old), a senior population (population of subjects who are 60 or older), and the like. The processing means **122** can form any population from a plurality of subjects and perform standardization processing on the first data set of the population.

For example, the processing means **122** can classify a first data set from a plurality of subjects into a data set for a male subject and a data set for a female subject and standardize the data set for a male subject to perform standardization processing on a male subject population, or standardize the data set for a female subject to perform standardization processing on a female subject population, or both. Standardization processing on a male subject population and/or female subject population in this manner is preferable for a parameter in a first parameter set with a difference between males and females (e.g., blood neural fat concentration or the like), and is more preferable for a parameter in a first parameter set with a significant difference between males and females (e.g., blood red blood cell count or the like). This is because this eliminates the difference between males and females and enables the creation of a health positioning map without a bias due to a difference between males and females.

Processing by the processing means **122** can include, for example, weighting processing. Weighting processing is processing for weighting at least some data in a first data set. For example, the processing can be configured to add weighting by adding a predetermine number to at least some data in a first data set, or by multiplying a predetermined number to at least some data in a first data set. The predetermined number that is added or multiplied can be constant or different for each data subjected to weighting processing. For example, a predetermined number can be varied so that larger or smaller weighting is added to data with a greater effect on a health function derived by the derivation means **133** described below, or alternatively, a predetermined number can be varied so that larger or smaller weighting is added to data with a lesser effect on a health function derived by the derivation means **133** described below.

The processing means **122** can be configured to perform weighting processing on a first data set for all of the plurality of subjects, or to perform weighting processing on a first data set for a specific population among the plurality of subjects. The processing means **122** can form any population from a plurality of subjects and perform weighting processing on a first data set of the population. A population subjected to weighting processing can be the same as or different from the aforementioned population subjected to standardization processing.

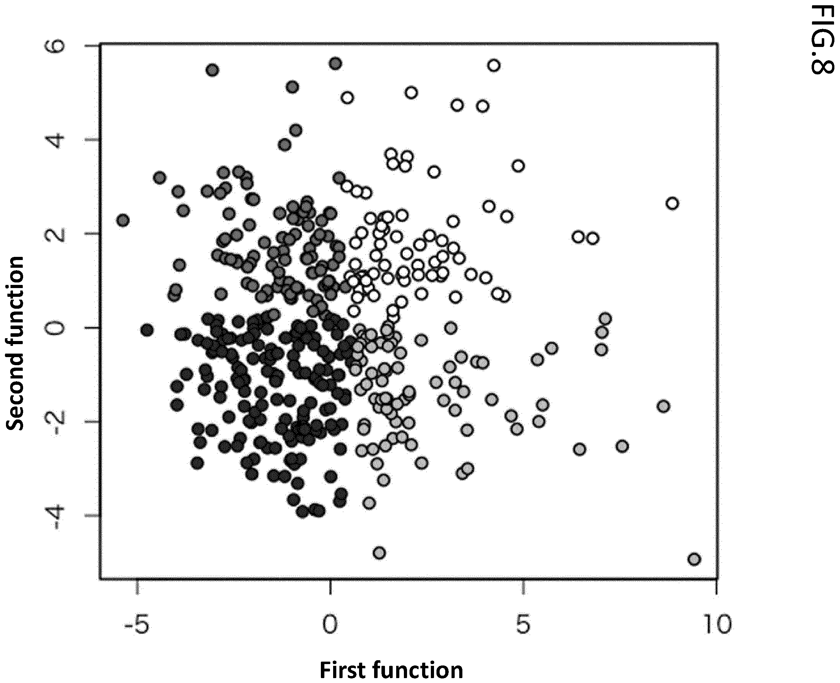

The mapping means **123** is configured to map an output of the processing means **122,** i.e., first data, for each of the plurality of subjects. Mapping by the mapping means **123** is processing that associates n-dimensional first data with a position on an n-dimensional space. The mapping means **123** can output a map mapping first data of each of the plurality of subjects by mapping the first data. If, for example, the first data is two dimensional, the mapping means **123** can output a two dimensional map by mapping first data so that the first data is associated with a position on a two dimensional space, i.e., plane. Figure **8** is a diagram showing an example of a result of mapping by the mapping means **123.** As shown in Figure **8****,** the mapping means **123** outputs a map by plotting points determined by first data obtained by the processing means **122** on a two dimensional space (multidimensional space) for the plurality of subjects. The mapping means **123** can be configured, for example, to output a map (radar map) which maps first data of each of a plurality of subjects by mapping n-dimensional first data onto a radar chart with n axes.

The clustering means **124** is configured to cluster first data mapped by the mapping means **123.** Clustering by the clustering means **124** is processing for dividing mapped first data into a plurality of clusters and identifying each region to which the plurality of clusters belong. As used herein, "region" refers to a certain range within an n-dimensional space, having an n-dimensional range. The clustering means **124** can divide mapped first data into any number of clusters. For example, the clustering means **124** preferably divides mapped first data into at least three clusters. This is because a health positioning map that is intuitively understandable by a user can be created by using three clusters (e.g., clusters such as good health level, normal health level, and poor health level as shown in Figure **1B****).** The number of clusters into which mapped first data is divided can be configured to be dependent on the number of the plurality of subjects **N.** In the example shown in Figure **8****,** the clustering means **124** classifies first data mapped by the mapping means **123** into four clusters. The clustering means **124** can cluster data using any known methodology. For example, the clustering means **124** can divide data into a plurality of clusters using a non-hierarchical clustering method (e.g., k-means, k-means ++, PAM, or the like). The clustering means **124** can preferably divide data into a plurality of clusters using k-means. This is because a result from clustering using k-means reflects the tendency of a subject population better and contains richer information, relative to a result of clustering by other methodologies. The clustering means **124** can, for example, identify a plurality of regions by defining a boundary that divides each of a plurality of clusters. Such defining of a boundary can be performed using any known processing. In the aforementioned example of a radar chart, the clustering means **124** can simply identify a plurality of axes as a plurality of regions by distinguishing a value of each axis from a value of another axis.

In one embodiment, the clustering means **124** can be configured to further cluster sub-first data acquired by the acquisition means **121.** The clustering means **124** can divide sub-first data into a plurality of clusters and identify each region to which the plurality of clusters belong. The clustering means **124** can divide sub-first data into any number of clusters.

The characterization means **125** is configured to characterize at least some of a plurality of regions identified by the clustering means **124.** The characterization means **125** can be configured, for example, to characterize at least some of a plurality of regions based on information inputted into the computer system **100** via the interface unit **110** by a health positioning map creator. For example, a health positioning map creator can analyze a characteristic of a subject corresponding to first data included in each of a plurality of regions and input information with which the regions should be characterized based on the result of analysis. Alternatively, the characterization means **125** can be configured to characterize at least some of a plurality of regions without depending on input by a health positioning map creator. For example, the characterization means **125** can characterize at least some of a plurality of regions based on a relative position on a health positioning map or based on machine learning.

In this manner, a health positioning map with at least some of the plurality of regions characterized is created. In one embodiment, a health positioning map from characterizing some of a plurality of regions identified by clustering sub-first data would be a health positioning map for some of the plurality of subjects.

A health positioning map created by the processing unit **120** is outputted, for example, to the outside of the computer system **100** via the interface unit **110.** A health positioning map can be transmitted, for example, to the database unit **200** via the interface **110** and stored in the database **200.** Alternatively, the map can be transmitted to the processing unit **130** described below for creating a health function. As described below, the processing unit **130** can be a constituent element of the same computer system **100** as the processing unit **120,** or a constituent element of another computer system.

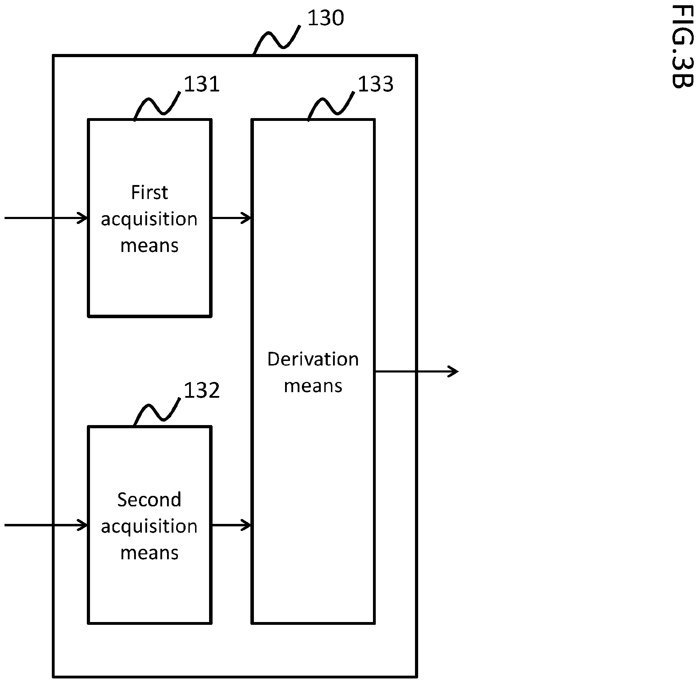

Figure **3B** shows an example of a configuration of the processing unit **130** in another embodiment. The processing unit **130** can have a configuration for processing that creates a health function for mapping a health level of a subject onto a health positioning map. The processing unit **130** can be a processing unit that the computer system **100** comprises in place of the aforementioned processing unit **120** or a processing unit that the computer system **100** comprises in addition to the processing unit **120.** If the processing unit **130** is a processing unit that the computer system **100** comprises in addition to the processing unit **120,** the processing unit **120** and the processing unit **130** can be implemented by the same processor or by different processors.

The processing unit **130** comprises first acquisition means **131,** second acquisition means **132,** and derivation means **133.**

The first acquisition means **131** is configured to acquire a health positioning map. The acquired health positioning map can be a health positioning map created by the aforementioned processing unit **120** or a health positioning map created in another manner, as long as the map is created using a first parameter set. The acquired health positioning map is passed along to the derivation means **133** for subsequent processing.

The second acquisition means **132** is configured to acquire a second data set with respect to a second parameter set described below for at least some of the plurality of subjects. A second parameter set is a part of the first parameter set. For example, the second acquisition means **132** can acquire data for some of a plurality of subjects stored in the database unit **200** via the interface unit **110.** The acquired second data set is passed along to the derivation means **133** for subsequent processing.

The derivation means **133** is configured to derive a health function that correlates a second data set acquired by the second acquisition means **132** with a position on a health positioning map acquired by the first acquisition means **131.** The derivation means **133** can derive a health function by, for example, machine learning, decision tree analysis, random forest regression, multiple regression analysis, principle component analysis, or the like. A health function can be derived, for example, for each axis of an n-dimensional health positioning map. If, for example, a health positioning map is two dimensional, a health function X for correlating a second data set with an X coordinate on the health positioning map and a health function Y for correlating the second data set with a Y coordinate on the health positioning map can be derived. The derivation means **133** can, for example, increase/decrease the number of variables of a health function to any number and create a plurality of health functions having the same degree of accuracy, i.e., health function group (hereinafter, also referred to as a multiple pattern heath function group). For example, the derivation means **133** can create (1) a health function using data for blood test items and data for other items as variables and (2) a health function using only data for blood test items as a variable, which has the same degree of accuracy to each other. The derivation means **133** can also create a health function group using data selected from a data group for items other than data for blood test items as a variable, as a multiple pattern health function group.

A health function can be, for example, a regression model. A regression model can be a linear regression model or a nonlinear regression model. The derivation means **133** can derive each coefficient of a regression model by machine learning using a second data set as an independent variable and a coordinate on a health positioning map of a subject as a dependent variable for each of at least some of the plurality of subjects. When a second data set obtained from a subject is inputted into such a machine learned regression model dependent variable, a coordinate on the health positioning map of the subject is outputted. The health level of the subject can be mapped onto the health positioning map by using the outputted coordinate.

A health function can be, for example, a neural network model. A neural network model has an input layer, at least one hidden layer, and an output layer. The number of nodes of an input layer of a neural network model corresponds to the number of dimensions of inputted data. Specifically, the number of nodes of an input layer corresponds to the number of parameters in a second parameter set. A hidden layer of a neural network model can comprise any number of nodes. The number of nodes of an output layer of a neural network model corresponds to the number of dimensions of outputted data. Specifically, when an X coordinate on a health positioning map is outputted from a neural network model, the number of nodes of an output layer would be 1. If, for example, n coordinates on an n-dimensional health positioning map are outputted from a neural network model, the number of nodes of an output layer would be n. The derivation means **133** can derive a weighting coefficient of each node by machine learning using a second data set as an input supervisor data and a position on the health positioning map of a subject as output supervisor data for each of at least some of the plurality of subjects.

For example, a set of (input supervisor data, output supervisor data) for machine learning can be (second data set with respect to a second parameter set for a first subject, coordinate on a health positioning map of a first subject), (second data set with respect to a second parameter set for a second subject, coordinate on a health positioning map of a second subject) ... (second data set with respect to a second parameter set for an ith subject, coordinate on a health positioning map of an ith subject) ... or the like. If a second data set obtained from a subject is inputted into an input layer of such a machine learned neural network model, a coordinate on a health positioning map of the subject is outputted to an output layer. A health level of the subject can be mapped onto a health positioning map.

A health function created by the processing unit **130** is outputted, for example, to the outside of the computer system **100** via the interface unit **110.** A health function can be transmitted, for example, to the database **200** via the interface **110** and stored in the database **200.** Alternatively, the function can be transmitted to the processing unit **140** described below for processing to estimate a health level of a user. As described below, the processing unit **140** can be a constituent element of the same computer system **100** as the processing unit **130** or a constituent element of another computer system.

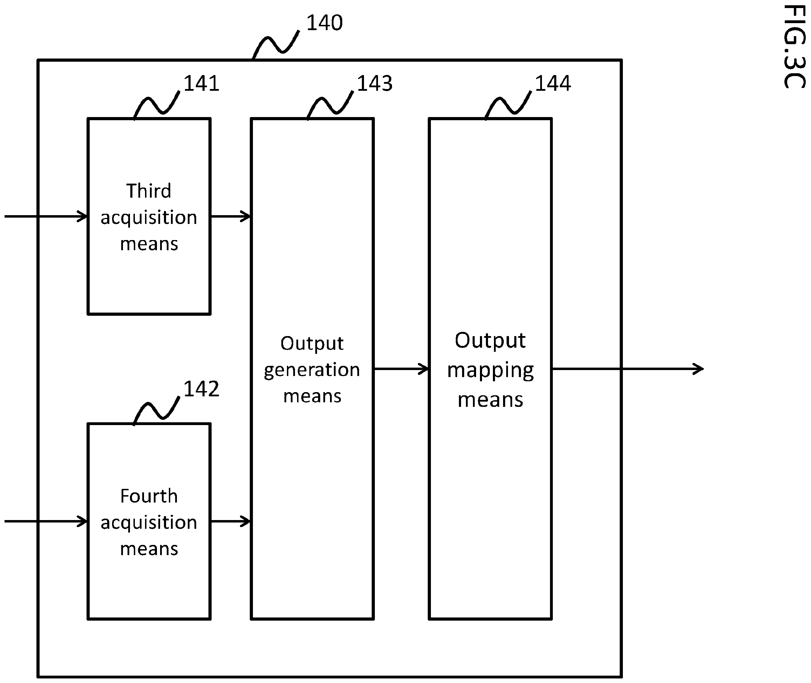

Figure **3C** shows an example of a configuration of the processing unit **140** in still another embodiment. The processing unit **140** can have a configuration for processing to estimate a health level of a user. Processing by the processing unit **140** can estimate the health level of a user by estimating which region on a health positioning map the health level of the user is positioned. The processing unit **140** can be a processing unit that the computer system **100** comprises in place of the aforementioned processing unit **120** and the processing unit **130** or a processing unit that the computer system **100** comprises in addition to the aforementioned processing unit **120** and/or processing unit **130.** If the processing unit **140** is a processing unit that the computer system **100** comprises in addition to the processing unit **120** and/or processing unit **130,** the processing unit **120,** the processing unit **130,** and the processing unit **140** can all be implemented by the same processor, all can be implemented by different processors, or two of the processing unit **120,** the processing unit **130,** and the processing unit **140** can be implemented by the same processor.

The processing unit **140** comprises third acquisition means **141,** fourth acquisition means **142,** output generation means **143,** and output mapping means **144.**

The third acquisition means **141** is configured to acquire a health function. A health function is a function that correlates a data set with respect to a second parameter set described above with a position on a health positioning map. The acquired health function can be a health function created by the aforementioned processing unit **130** or a health function created in another manner, as long as the function can correlate a user data set with a position on a health positioning map. The health positioning map can be a health positioning map created by the aforementioned processing unit **120** or a health positioning map created in another manner, as long as the map is created using a first parameter set. The acquired health function is passed along to the output generation means **143** for subsequent processing.

The fourth acquisition means **142** is configured to acquire a user data set with respect to a second parameter set of a user. The fourth acquisition means **142** can acquire, for example, a user data set stored in the database unit **200** via the interface unit **110.** Alternatively, the fourth acquisition means **142** can acquire, for example, a user data set via the interface unit **110** from a user terminal apparatus. The acquired user data set is passed along to the output generation means **143** for subsequent processing.

The output generation means **143** is configured to generate an output from a health function. The output generation means **143** generates an output from a health function by inputting a user data set acquired by the fourth acquisition means **142** into a health function acquired by the third acquisition means **141.**

If, for example, a health function is a regression model as described above, a coordinate on a health positioning map is outputted by inputting a user data set into an independent variable of the regression model.

If, for example, a health function is a neural network model as described above, a coordinate on a health positioning map is outputted by inputting a user data set into an input layer of the neural network model.

The output mapping means **144** is configured to map an output generated by the output generation means **143** onto a health positioning map. Since an output generated by the output generation means **143** is a coordinate, the output mapping means **144** can map the coordinate within an n-dimensional space of a health positioning map.

An output mapped onto a health positioning map by the processing unit **140** is outputted, for example, to the outside of the computer system **100** via the interface unit **110.** An output can be transmitted, for example, to a terminal apparatus of a user via the interface unit **110.**

Each of the aforementioned constituent elements of the computer system **100** can be comprised of a single hardware part or a plurality of hardware parts. If comprised of a plurality of hardware parts, the mode of connecting each hardware part is not limited. Each hardware part can be connected wirelessly or with a wire. The computer system **100** of the invention is not limited to a specific hardware configuration. The processing units **120, 130,** and **140** comprised of analog circuits instead of digital circuits are also within the scope of the invention. The configuration of the computer system **100** of the invention is not limited to those described above, as long as the function thereof can be materialized.

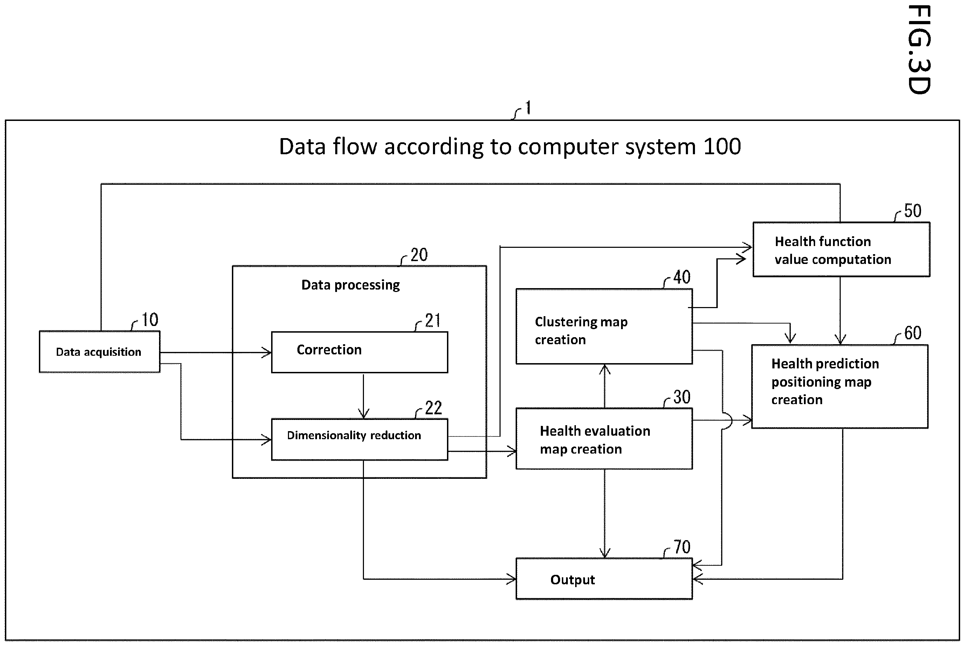

Figure **3D** is a diagram showing an example of data flow **1** according to the computer system **100** in one embodiment. As shown in Figure **3D****,** the data flow **1** comprises a data acquisition step **10,** a data processing step **20,** a health evaluation map creation step **30,** a clustering map creation step **40,** a health function value computation step **50,** a positioning map creation step **60,** and an output step **70.** For example, the data acquisition step **10,** the data processing step **20,** the health evaluation map creation step **30,** and the clustering map creation step **40** have a function as a health positioning map creation apparatus, which can be implemented, for example, by the computer system **100** comprising the aforementioned processing unit **120.** The data acquisition step **10** and the health function value computation step **50** have a function as a health function value computation apparatus, which can be implemented, for example, by the computer system **100** comprising the aforementioned processing unit **140.** At the output step **70,** data generated at the data processing step **20,** the health evaluation map creation step **30,** the clustering map creation step **40,** the health function value computation step **50,** or the positioning map creation step **60** is outputted. The data is outputted, for example, by a display apparatus (e.g., liquid crystal display).

A data set acquired at the data acquisition step **10** is send to the data processing step **20.**

The data processing step **20** performs, for example, correction **21** and dimensionality reduction **22** as shown in Figure **3D****.** The data processing step **20** can be implemented, for example, by the processing means **122** of the aforementioned processing unit **120.**

The correction **21** is correction of data acquired at the data acquisition step **10.** Specifically, the correction **21** corrects each data acquired at the data acquisition step **10** so that a value of the data acquired at the data acquisition step **10** is, for example, a value within a predetermined range (e.g., mean value is 0 and standard deviation is 1). Corrected data is send to the dimensionality reduction **22.**

The dimensionality reduction **22** reduces the dimension of a plurality of pieces of data passed along from the data acquisition step **10** or the correction step **21.** Specifically, the dimensionality reduction **22** reduces the dimension of a plurality of pieces of data (multidimensional data) sent from the data acquisition step **10** or the correction step **21** to any dimension (two dimension in this embodiment) using multiple regression analysis, multidimensional scaling, principle component analysis, or machine learning. For example, data with reduced dimensionality can be in a form of a function. For example, this function is a function for computing an indicator associated with health. A function is, for example, a function using some or all data contained in first data as a variable, and is a function created by placing a greater weighting on data with a particularly significant effect among various disease factors. A function in this embodiment is created using some or all of data contained in first data using a linear or nonlinear model. In one embodiment, two dimensional function (in this embodiment, comprised of horizontal axis function X (hereinafter, referred to as a first function) and vertical axis function Y (hereinafter, referred to as a second function)) is created. The created function is passed along to the health evaluation map creation step **30** and the output step **70.**

In this embodiment, the dimensionality reduction **22** creates a two dimensional function by reducing multidimensional data to two dimensions as described above. The first function and second function are functions using all or some of a plurality of pieces of data passed along from the data acquisition step **10** or the correction step **21** as a variable, and are functions computed by multiple regression analysis, multidimensional scaling, principle component analysis, or machine learning. In the present invention, "machine learning" refers to either machine learning with deep learning or machine learning without deep learning. The first function and the second function can have completely identical or completely different constituent variables, or some variables can overlap with each other.

The health evaluation map creation step **30** can be implemented by, for example, the mapping means **123** of the aforementioned processing unit **120.** At the health evaluation map creation step **30,** a map for evaluation health (hereinafter, referred to as a health evaluation map) is created using, for example, data processed by the data processing step **20,** more specifically a function created by dimensionality reduction. In this embodiment, a function is two-dimensional, so that a health evaluation map is a two-dimensional map. Figure **8** is a diagram showing an example of a health evaluation map. As shown in Figure **8****,** a health evaluation map is created by plotting points determined by the first function and second function in a two-dimensional space (multidimensional space) for a plurality of subjects in the health evaluation map creation step **30.**

The clustering map creation step **40** can be implemented by, for example, the clustering means **124** and the characterization means **125** of the aforementioned processing unit **120.** For example, a map from clustering a plurality of points plotted on a health evaluation map created at the health evaluation map creation step **30** into several clusters and characterizing a plurality of regions (hereinafter, referred to as a health positioning map) is created at the clustering map creation step **40.** At the clustering map creation step **40** in this embodiment, a plurality of plotted points are clustered into any number of clusters using a nonhierarchical clustering method (k-means in this embodiment). In the example shown in Figure **8****,** points are classified into four clusters. A health positioning map is created by characterizing at least one of the four regions with information related to health.

A created health positioning map can be outputted at the output step **70,** passed along to the health function value computation step **50,** or passed along to the health prediction positioning map creation step **60.**

The health function value computation step **50** and the health prediction positioning map creation step **60** can be implemented by, for example, the aforementioned processing unit **130** and processing unit **140.** At the health function value computation step **50,** a health function is first created based on a health positioning map. The number of variables of a health function can be optionally increased/decreased to create a plurality of health functions with the same degree of accuracy, i.e., a health function group. A value of a health function of a subject who is different from the subject from whom a first data set was acquired to create a health function is then computed. Specifically, a value of a health function of a subject is computed by applying the created health function on data of the subject acquired at the data acquisition step **10** (newly acquired data). Since a health function is a multidimensional function, a health function value is naturally a multidimensional value.

The health prediction positioning map creation step **60** creates a health prediction positioning map by plotting a value of a health function of a subject computed by the health function value computation step **50** on a health evaluation map created by the health evaluation map creation step **30** or a health positioning map created by the clustering map creation step **40.** This enables prediction of a health status of a subject from whom data was newly measured.

The health prediction positioning map creation step **60** can also create a health prediction positioning map by plotting a value of a health function computed at the health function value computation step **50** using data acquired with a time interval on the health evaluation map or the health positioning map described above for a single subject. This enables evaluation of a degree in change of the health status of the subject.

Newly acquired data from a subject can also be used as data for updating a health function. At the health function value computation step **50,** a health function can be updated by further using newly acquired data from a subject or data outputted from the correction step **21** using said data. This enables evaluation of a greater variety of health risks and increase in the accuracy of health risk evaluation.

Each step of data flow **1** (especially the data processing step **20,** health evaluation map creation step **30,** clustering map creation step **40,** health function value computation step **50,** and health prediction positioning map creation step **60)** can be materialized with a logic circuit (hardware) formed into an integrated circuit (IC chip) or the like, or with a software. For the latter, the heath evaluation apparatus **1** comprises a computer for executing an instruction of a program, which is software materializing each function. Such a computer comprises, for example, one or more processors, and a computer readable recording medium for storing the program described above. The objective of the invention is accomplished by the processor reading out the program from the recording medium and executing the program in the computer. For example, a CPU (Central Processing Unit) can be used as the processor. As the recording medium, a "non-transient tangible medium" such as ROM (Read Only Memory) and the like, as well as tape, disk, card, semiconductor memory, programmable logic circuit, and the like can be used. This can also further comprise a RAM (Random Access Memory) or the like for deploying the program. The program can also be supplied to the computer via any transmission medium (communication network, broadcast wave, or the like) that is capable of transmitting the program. In one embodiment of the invention, the program can be materialized in a form of a data signal embedded into a carrier wave, actualized by electronic transmission.

According to the data flow described above, a health positioning map and/or health function can be created using various data. Specifically, a health positioning map and/or health function that can comprehensively compute the health level of a subject, instead of an existing health indicator associated with an individual disease, can be created. Therefore, a health positioning map and/or health function that is capable of evaluating various health risks (i.e., capable of evaluating the overall health level of a subject) can be created.

A health positioning map and/or health function can also be flexibly made by combining various data measured at the data acquisition step **10.** Specifically, the versatility in the selection of items measured at the data acquisition step **10** is very high.

In one embodiment, a health function is created by machine learning using a second data set as input data. This enables the creation of a health function which is capable of making an indicator of a health status of a subject more accurately.

In one embodiment, data is corrected between sexes before creating a health positioning map with a first data set. This enables the creation of a health positioning map that is compatible for either sex.

In one embodiment of the invention, first data can be in a form comprising only data acquired by noninvasive measurement. Such a configuration can acquire data without injuring a subject through a blood test or the like.

In one embodiment, a map for evaluating health can be created by using a function created at the data processing step **20.** Specifically, if a function is a multidimensional vector, a map for evaluating health is created by plotting points determined by a function into a multidimensional space for a plurality of subjects. This enables visual inspection of the health status of subjects.

In one embodiment, points plotted in a multidimensional space are clustered. By referring to clustered data on subjects belonging to each cluster, the type of subject population can be identified for each cluster to characterize the clusters. As a result, the health status of a measured subject can be predicted by plotting newly measured data of the subject on a health positioning map.

In the example shown in Figure **2****,** the database unit **200** is provided external to the computer system **100,** but the present invention is not limited thereto. At least a part of the database unit **200** can also be provided inside the computer system **100.** In such a configuration, at least a part of the database unit **200** can be implemented by the same or different storage means as the storage means implementing the memory unit **150.** In either configuration, at least a part of the database unit **200** is configured as a storage unit for the computer system **100.** The configuration of the database unit **200** is not limited to a specific hardware configuration. For example, the database unit **200** can be comprised of a single hardware part or a plurality of hardware parts. For example, the database unit **200** can be configured as an external hard disk drive of the computer system **100,** or as a storage on the cloud connected via a network **400.**

### 3. First parameter set

The inventors focused on physiological or biochemical mechanisms shared by deterioration in health and chronic fatigue in order to evaluate the overall health level of a subject. Although not wishing to be bound by any theory, there can be common mechanisms among fatigue to chronic fatigue, deterioration in health, senescence, and disease development based on medical research on fatigue and follow up study on deterioration in health. The common mechanisms are:
(1) "biological oxidation (rusting)" indicating progression of biological oxidation and a reduced antioxidant capability;
(2) "less repair energy" indicating delayed recovery from the aforementioned biological oxidation
(3) "inflammation" indicating that an oxidated (rusted) cell is detected; and
(4) "autonomic nerve function" indicating a function for sensing and regulating (1) to (3).
Comprehensive evaluation of the parameters (1) to (4) enables the determination of a state of deteriorated health (i.e., "ahead sick") that has not developed into a disease.

In a representative embodiment, a first parameter set for acquiring a first data set in the present invention can comprise a "biological oxidation parameter", "less repair energy parameter", "inflammation parameter", and "autonomic nerve function parameter", based on the theory described above. As used herein, the four parameters, i.e., (1) "biological oxidation parameter", (2) "less repair energy parameter", (3) "inflammation parameter", and (4) "autonomic nerve function parameter", are also collectively referred to as the four basic parameters.

### (Biological oxidation parameter)

Reactive Oxygen Species (ROS) denature many biopolymers constituting a cell such as DNAs, lipids, proteins, and enzymes in the body by oxidation, thus damaging cellular functions. Denaturation by oxidation due to reactive oxygen species is understood to lead to various diseases and senescence.

Meanwhile, antioxidant enzymes such as superoxide dismutase (SOD) and catalase and antioxidants such as CoQ₁₀, vitamin C, and vitamin E are present in the body in order to prevent cellular dysfunction due to reactive oxygen species.

Therefore, measurement of "biological oxidation parameter" in the present invention can comprise measurement of oxidative damage due to reactive oxygen species, measurement of antioxidant capability, or measurement of the balance between oxidative damage and antioxidant capability. Oxidative damage due to reactive oxygen species can be measured by directly measuring the amount of reactive oxygen species, or by measuring oxidative damage on proteins, lipids, or nucleic acids.

A method of measuring oxidative damage is well known in the art. Those skilled in the art can appropriately select and measure a subject of measurement. In the present invention, examples of specific markers used as an indicator for oxidative damage due to reactive oxygen species include, but are not limited, d-ROMs (Derivatives of Reaction Oxygen Metabolites) that directly measure the amount of reactive oxygen species in blood, carbonylated protein content (PCC; Protein Carbonyl Content), which is an indicator of oxidative damage on proteins, 4-hydroxynonenal and isoprostane, which are indicators of oxidative damage on lipids, 8-OHdG (8-hydroxy-dioxyguanosine), which is an indicator of oxidative damage on nucleic acids, and the like.

A method of measuring an antioxidant capability is well known in the art. Those skilled in the art can appropriately select and measure a subject of measurement. In the present invention, examples of specific markers used as an indicator for antioxidant capability include, but are not limited to, BAP (Biological Antioxidant Potential), which quantifies the ability to reduce to iron, serum thiol status, glutathione measurement, vitamin C content measurement, total CoQ₁₀ content, ratio of reduced form of CoQ₁₀, and the like. The total CoQ₁₀ content and ratio of reduced form of CoQ₁₀ can be measured by, for example, LC-MS/MS (specific example can include computing from the concentration of reduced or oxidized CoQ₁₀ detected by multiple reaction monitoring).

In the present invention, examples of a marker used as an indicator for the balance between oxidative damage and antioxidant capability include, but are not limited to, OSI (Oxidation Stress Index). The OSI in the present invention is d-ROMs/BAP.

Preferred biological oxidation parameters in the invention include BAP, total CoQ₁₀ content, ratio of reduced form of CoQ₁₀, and OSI.

Typically, a biological oxidation parameter is a parameter that can be measured by a noninvasive test.

### (less repair energy parameter)

Even if a biological tissue is damaged by oxidation, the body is equipped with a mechanism for repairing the damaged tissue. ATP is required for tissue damage in the body. However, reduced ATP production would delay the repair of the damaged tissue. Reduced ATP production also leads to delayed recovery from fatigue. The "less repair energy parameter" in the present invention is any parameter indicating that ATP production is reduced.

ATP is produced via glycolysis, TCA cycle, and electron transport chain, but the largest amount of ATP is produced at the last electron transport chain. CoQ₁₀ is a coenzyme with a critical role in the electron transport chain. Thus, examples of the "less repair energy parameter" in the invention include, but are not limited to, total CoQ₁₀ content, ratio of reduced form of CoQ₁₀, and metabolites of glycolysis or TCA cycle (e.g., pyruvic acid, lactic acid, citric acid, isocitric acid, succinic acid, fumaric acid, malic acid, and the like). It should be noted that the total CoQ₁₀ content and ratio of reduced form of CoQ₁₀ are a "biological oxidation parameter" due to having an antioxidant capability as well as a "less repair energy parameter" due to contributing to ATP production. The preferred "less repair energy parameter" in the invention can include the total CoQ₁₀ content and ratio of reduced form of CoQ₁₀.

The method of measuring the total CoQ₁₀ content or ratio of reduced form of CoQ₁₀ is described above. A metabolite of glycolysis or TCA cycle can be measured by extracting a compound reflecting glycolysis or TCA cycle from metabolone analysis.

Typically, a less repair energy parameter is a parameter that can be measured by a noninvasive test.

### (Inflammation parameter)

Many tissue damages due to oxidation in the body result in many instances of local inflammation due to immune responses. The type of inflammation parameter and measurement method thereof are known in the art. Those skilled in the art can suitably select and measure an inflammation parameter.

Examples of the inflammation parameter in the invention include, but are not limited to, CRP (C-Reactive Protein), WBC (white blood cell count), albumin, red blood cell count, interleukin-1β, interleukin-6, and the like.

Typically, an inflammation parameter is a parameter that can be measured by a noninvasive test.

### (Autonomic nerve function parameter)

If deterioration in health is closely inspected in chronological order, the autonomic nerve function (especially the parasympathetic nerve function) initially decreases, then the quality of sleep decreases, then fatigue accumulates, and loss of motivation, depressive tendency, immune system malfunction such as allergies, endocrine system abnormality such as irregular menstruation, digestive system abnormality, or the like is observed. Therefore, an abnormality in the autonomic nerve function is an important parameter for the understanding of the initial stage of deterioration in health.

In the present invention, an autonomic nerve function is evaluated from a heartbeat parameter. Specific examples of the heartbeat parameter used in the invention include but are not limited to the following.

### *Mean HR

This refers to the mean value of total heartbeat count in 5 minutes.

### *Activity of the entire autonomic nervous system; TP (Total Power = ms²)

This is the computed value of total power of a power spectrum at a frequency of 0 to 0.4 Hz (VLF, LF, HF) in a measurement of 5 minutes. This value reflects the overall activity of the autonomic nervous system primarily accounted for by the activity of sympathetic nerves and is understood as a numerical value associated with fatigue.

### *Overall activity of the sympathetic nervous function; VLF (very low frequency ms²)

This is the power spectrum at the frequency band of about 0.0033 to 0.04 Hz. Generally, this parameter is understood to indicate the overall activity of a very slow mechanism of a sympathetic nerve function.

### *Activity of sympathetic nerve; LF (lower frequency ms²)

This is the power spectrum at the frequency band of about 0.004 to 0.15 Hz. This value primarily reflects the activity of (vasomotor) sympathetic nerve.

### *Activity of parasympathetic nerve; HF (high frequency ms²)

This is the power spectrum at the frequency band of about 0.15 to 0.4 Hz. This value reflects the parasympathetic nerve (vagus nerve) activity.

### *Balance between a sympathetic nerve and a parasympathetic nerve; LF/HF ratio

This is the ratio of power of LF (lower frequency) to HF (high frequency). This value represents the overall balance of the sympathetic nerve and the parasympathetic nerve. Generally, a higher numerical value indicates sympathetic nerve dominance, and a lower value indicates parasympathetic nerve dominance.

The heartbeat parameter described above representing the autonomic nerve function can be measured by a method known in the art, but can also be measured by an accurate methodology for simultaneously measuring electrocardiographic waves and plethysmographic waves to analyze variation in heartbeats (see Japanese Patent No. 5455071 and Japanese Patent No. 5491749, which are incorporated herein by reference). For example, the autonomic nerve function of the invention can be measured by using a simplified autonomic nervous system measuring apparatus FMCC-VSM301 (Fatigue Science Laboratory Inc., Osaka, Japan) that can simultaneously measure electrocardiographic waves and plethysmographic waves.

The preferred "autonomic nerve function parameter" in the invention can be, but is not limited to, mean HR, TP, LF, HF, LF/HF, or the like. Since the values of HF and LF have large dispersion that is not distributed under a normal distribution, it is preferable to evaluate TP, LF, HF, and LF/HF associated with HF or LF among the parameters described above by applying logarithmic conversion. Therefore, the more preferred "autonomic nerve function parameter" in the invention includes means HR, ln(TP), ln(LF), ln(HF), and ln(LF/HF).

Typically, an autonomic nerve function parameter is a parameter that can be measured by a noninvasive test.

### (Additional first parameter set)

The first parameter set of the invention can comprise one or more of the following parameters in order to create a positioning map that more suitably evaluates the overall health level of a subject.

### *Fundamental parameter

Fundamental parameters include known parameters representing the physical condition or health status of a subject. The fundamental parameters of the invention include, but are not limited to, age, height, boy weight, waist circumference, body composition, bone density, blood pressure, muscle strength, and the like.

Body composition includes, but is not limited to, muscle mass, BMI (body weight/height = Body Mass Index), fat percentage, and the like.

A method of measuring bone density is known, such as the MD method for capturing an image of a boned of a hand and measuring the bond density from the picture, ultrasound method for measuring the heel bone using ultrasound waves, QCT using CT scan, and DEXA (Dual energy X-ray absorptiometry) using X-rays and a computer. A parameter obtained from any of the measuring methods can be used in the present invention.

Examples of the bone density parameters in the invention include, but are not limited to, Speed of Sound (SOS), Osteoporosis Index (e.g., OSIRIS (Osteoporosis Index of Risk)), young adult comparative % (YAM = Young Adult Mean; % of BMD (Bone Mineral Density; bone density = bone mass/area (unit: g/cm²) value of subject in comparison to BMD value of young individuals when BMD value of young individuals (reference value) is assumed to be 100%), T score (value from defining an indicator with mean BMD value (reference value) of young individuals as 0 and standard deviation as 1 SD), and the like. Methods of measuring these fundamental parameters are well known in the art.

Blood pressure is conventionally measured in the art. Systolic blood pressure can be used as the fundamental parameter of the invention.

Muscle strength is conventionally measured in the art. Muscle mass and mean grip strength of left and right hands can be used as the fundamental parameter of the invention.

The fundamental parameters of the invention can preferably comprise age, muscle mass, BMI, fat percentage, SOS, OI, systolic blood pressure, and mean grip strength of left and right hands.

A fundamental parameter is a parameter that can be measured by a noninvasive test.

### *Hematological parameter

It is preferable that a commonly used hematological parameter is further included in a first parameter set in order to evaluate renal excretion/liver, gallbladder, and pancreas/detoxification function system of a subject in addition to the biological oxidation parameters described above.

Examples of such hematological parameters include, but are not limited to, HbA1c (hemoglobin A1c; glycated protein from glucose binding to hemoglobin), ALP (alkaline phosphatase), ALT (alanine aminotransferase), AST (aspartate aminotransferase), BS (blood sugar level), BUN (blood urea nitrogen), CK (creatine kinase; plasma muscle cell enzyme that can be used in the evaluation of motor/skeletal/muscle function system), G-GT (gamma-glutamyl transpeptidase), HDL-C (HDL-cholesterol), HGB (hemoglobin), LD (lactate dehydrogenase), LDL-C (LDL cholesterol), TG (triglyceride; neutral fat), T-P (total protein), UA (uric acid), amylase, albumin, potassium, creatinine, chlorine, cortisol, sodium, eGFR, vitamin (e.g., vitamin B1), mineral (iron, copper, calcium, etc.) content, and the like.

Typically, a hematological parameter is a parameter that can be measured by an invasive test.

### *Cognitive function parameter

According to the studies of the inventors, accumulation of fatigue leads to decreased cognitive function. Therefore, the first parameter of the invention can further comprise a cognitive function parameter.

The cognitive function parameter of the invention can be obtained by TMT (Trail Making test), which is a simple cognitive function test of tracing indicators such as numbers from 1 to 25 written on a piece of paper with a pencil in order, ATMT (Advanced Trail Making Test) for performing TMT on a touch panel, modified ATMT developed by the inventors (K. Mizuno et al. Brain & Development 33 (2011) 412-420), or the like. Although TMT, ATMT, and modified ATMT have differences in methodology, subjects of measurement are the same, which are all cognitive function parameters of the invention.

As modified ATMT, the inventors prepared, for example, five problems for evaluating various elements of cognitive function, which can be used alone or in combination. Each of the cognitive problems can be evaluated by total reaction time or total number of total number of correct answers.

A cognitive function parameter is a parameter that can be measured by a noninvasive test.

### *Blood vessel and skin parameter

According to the studies conducted by the inventors, accumulation of fatigue can affect the blood vessel and skin. Therefore, the first parameter set of the invention preferably can comprise a blood vessel and skin parameter.

Examples of blood vessel parameters include, but are not limited to, blood vessel age, mean value of capillary length, cloudiness of blood vessel, number of blood vessels, and the like. The mean value of capillary length, cloudiness of blood vessel, and number of blood vessels can preferably be readily measured by image processing on the capillary course at the nail bed of the finger. Image processing on the capillary course and measurement of these parameters can be performed with, for example, a capillary scope manufactured by At Co., Ltd. (Osaka, Japan).

Examples of skin parameters include, but are not limited to, moisture content in the skin of an arm, amount of moisture evaporation, gloss, and the like. Methods of measuring moisture content in the skin of an arm, amount of moisture evaporation, and gloss are well known in the art.

A blood vessel and skin parameter is a parameter that can be measured by a noninvasive test.

### *Subjective evaluation of parameter

The first parameter set of the invention can also comprise a subjective evaluation parameter of a subject in addition to an objective parameter obtained by the measurement described above. Statuses such as physical, fatigue, and mental states of a subject that cannot be sufficiently understood from measurement of various components can be reflected in a health positioning map by adding subjective evaluation to a parameter set in addition to objective parameters from various measurement values.

In one embodiment, the subjective evaluation parameter in the invention can comprise subjective evaluation on fatigue, sleep, or mental state.

Subjective evaluation of fatigue can comprise one or more of subjective evaluation of fatigue duration, question related to a disorder due to fatigue, fatigue VAS (Visual Analogue Scale), Chalder Fatigue Scale (CFQ), fatigue symptom score computed using 11 items in CFQ (CFQ 11) (Tanaka M et al., Psychol Rep_2010, 106, 2, 567-575), questionnaire or VAS related to presenteeism, and questionnaire related to fatigue. "Question related to a disorder due to fatigue" refers to a question that checks subjective evaluation of a subject with respect to presence/absence of a cause and effect relationship between fatigue and some type of a disorder. "Question related to a disorder due to fatigue" can be, for example, a question on a disease thought to be the cause of fatigue, whether the subject feels that fatigue is impeding with work, household chores, or school work, or the like. "Questionnaire related to fatigue" asks whether the subject is aware of any symptom of fatigue, which can be, for example, whether the subject feels lethargy, whether the subject feels that fatigue remains even after a night of sleep, or the like. For example, WHO's Health and Work Performance Questionnaire (HPQ), Work Limitations Questionnaire (WLQ), or the like can be used as a questionnaire on presenteeism.

Subjective evaluation of sleep can comprise one or more of time of sleep (sleep and wake time), mean sleep time, VAS related to drowsiness, and questionnaire related to the quality of sleep.

Subjective evaluation of mental state can include one or more of VAS and questionnaire related to depression, and VAS and questionnaire related to enthusiasm. "Questionnaire related to depression" refers to questions related to any symptom of depression, which can include whether the subject feels melancholy, whether the subjects feels tiresome about interacting with others, or the like. One example thereof is a K6 total score (indicator commonly used as an indicator representing a mental problem developed by Kessler et al.)

The "questionnaire" described above can be evaluated by a response to a specific question or evaluated by converting responses to a large number of questions into a score.

A subjective parameter is a parameter that can be measured by a noninvasive test.

### *Living condition parameter

The first parameter set of the invention can comprise a living condition parameter in addition to an objective parameter and subjective evaluation parameter. The living condition parameter in the invention is a fact about the living conditions of a subject. Examples thereof can include years of education, period of marriage, presence/absence of cohabitant, smoking (yes/no, frequency, and/or amount), drinking (yes/no, frequency, and/or amount), working hours, exercise (yes/no, frequency, and/or amount), meals (whether the subject feels meals are frequent or early, frequency of meals after dinner, frequency of skipping breakfast, etc.), medical history, drug dosing, supplement dosing, and the like.

A living condition parameter is a parameter that can be measured by a noninvasive test.

### *Additional parameter

The first parameter set of the invention can include a parameter based on data associated with cerebral function/neuropsychiatric system evaluation, circulatory/respiratory function system evaluation, or renal excretion/liver, gallbladder, and pancreas/detoxification function system evaluation.

For example, data associated with cerebral function/neuropsychiatric system evaluation can comprise data such as communication function, amount of activity (during the day or during sleep), brain anatomy measurement by MRI (Magnetic Resonance Imaging) (can measure decrease in function corresponding to a contracted site of brain tissue), fMRI at rest, and nerve fiber bundle course anisotropy (size and durability of nerve fiber bundle), in addition to those described above.

Data associated with circulatory/respiratory function system evaluation can include blood flow volume (can be measured, for example, with a Doppler blood flow meter), expirated gas component analysis (NO (asthma), acetone (diabetes), or the like), or the like, in addition to those described above. Data associated with expirated gas component analysis can be measured by mass spectrometry or ion mobility spectrometry.

Data associated with renal excretion/liver, gallbladder, and pancreas/detoxification function system evaluation can include data from skin gas component analysis or the like in addition to those described above. Data associated with skin gas component analysis can be measured by mass spectrometry or a high sensitivity variable laser detector.

### (Preferred first parameter set)

In one embodiment, the first parameter set of the invention can comprise a biological oxidation parameter, less repair energy parameter, inflammation parameter, and autonomic nerve parameter (four basic parameters). By creating a health positioning map based on data with respect to a first parameter set including the four basis parameters, the health positioning map enables determination of a deteriorated health state (i.e., "ahead sick") that has not resulted in a disease.

In another embodiment, the first parameter set of the invention can comprise four basic parameters, a fundamental parameter, a cognitive function parameter, and a subjective parameter. Although not wishing to be bound by any theory, it is understood that a wide ranging fatigue or mental states can be evaluated by adding a fundamental parameter, a cognitive function parameter, and a subjective parameter in addition to the four basic parameters. A subjective parameter preferably comprises evaluation on one or more of fatigue, sleep, and mental state, and more preferably comprises at least evaluation on fatigue.

In still another embodiment, the first parameter set of the invention can comprise four basic parameters, a fundamental parameter, a cognitive function parameter, a subjective parameter, and a hematological parameter. Although not wishing to be bound by any theory, a wide ranging health levels comprising additional different viewpoint can be evaluated by further adding a hematological parameter that can accurately evaluate a function of the endocrine system to the first parameter set, in addition to the four basic parameters, and a fundamental parameter, cognitive function parameter, and subjective parameter for evaluating fatigue or mental state.

In still another embodiment, the first parameter set of the invention can comprise four basic parameters, a fundamental parameter, a cognitive function parameter, a subjective parameter, a hematological parameter, a blood vessel and skin parameter, and a living condition parameter.

The first parameter set of the invention typically comprises both an invasive parameter and a noninvasive parameter. This would reflect information on an invasive parameter in a positioning map which is the base of evaluation, even when the second parameter set described below is comprised of only noninvasive parameters. As a result, evaluation including the effect of a parameter that can be obtained only through an invasive test can be performed by testing with only a noninvasive parameter. This is a significant effect of the inventions of the present application.

### 4. Second parameter set

In one embodiment, a second parameter set can comprise the following.
(1) age;
(2) fat percentage;
(3) neutral fat (TG);
(4) CRP;
(5) OSI;
(6) subjective evaluation on fatigue;
(7) balance of autonomic nerve (e.g., LF/HF or logarithmic value thereof);
(8) a cognitive function.

In a preferred embodiment of the invention, a second parameter set can be comprised of only noninvasive parameters. However, a positioning map for evaluating the second parameter set can be a map created by including an invasive parameter, so that evaluation that essentially comprises also the effect of an invasive parameter can be performed by evaluating the overall health of a subject using the second parameter set comprised of only noninvasive parameters. This is a significant effect of the invention. Further, estimation of a health level of a user from such a parameter set that does not comprise an invasive test result enables a user to conveniently find the user's own health level, regardless of the testing site. A user can find the user's own health level from a simple test performed, for example, at a company, drug store, community center, cafe, home, and the like in addition to hospitals.

A second parameter set comprised of only noninvasive parameters can comprise the following:
(1) age;
(2) BMI;
(3) fat percentage;
(4) SOS;
(5) systolic blood pressure;
(6) subjective evaluation on fatigue;
(7) subjective evaluation on depression;
(8) activity of parasympathetic nerve (e.g., HF or a logarithmic value thereof);
(9) activity of an entire autonomic nervous system (e.g., TP or a logarithmic value thereof); and
(10) cognitive function.

### 5. Processing by computer system

Figure **5A** shows an example of processing in the computer system **100.** Processing **500** is processing for creating a health positioning map. The processing **500** is executed in the processing unit **120** of the computer system **100.**

At step **S501,** the acquisition means **121** of the processing unit **120** acquires a first data set with respect to a first parameter set for each of a plurality of subjects. The acquisition means **121** can acquire data, for example, by receiving data on the plurality of subjects stored in the database **200** via the interface unit **110.** The acquisition means **121** can acquired data, for example, by receiving data on the plurality of subjects stored in the database unit **200** from the interface unit **110** from a computer system of a test facility (e.g., hospital, research laboratory, or the like).

At step **S502,** the processing means **122** of the processing unit **120** can obtain first data by processing a first data set acquired at step **S501.** Processing by the processing means **122** can comprise, for example, at least one of dimensionality reduction processing, standardization processing, and weighting processing on the first data set.

Preferably, processing by the processing means **122** comprises dimensionality reduction processing on the first data set. This is because such processing can reduce the dimension of a multidimensional and complex first data set to a more readily understandable data and consequently a health positioning map. At this time, dimensionality reduction processing preferably reduces the first data set to two dimensional data or three dimensional data. This is because a health positioning map created from two dimensional data or three dimensional data would be a map of a two-dimensional space or a three-dimensional space and visually readily understandable. By performing dimensionality reduction processing, (1) contribution of each measurement item to a value of each axis of a health positioning map can be found, and (2) data on a health positioning map can be more clearly understood by excluding data for a measurement item that is not significantly associated with a value of each axis of a health positioning map.

More preferably, processing by the processing means **122** comprises standardization processing on a first data set and dimensionality reduction processing on the standardized data set. This is because this eliminates a scalar difference between parameters of a first data set, and the effect of each parameter of the first data set on the health positioning map is considered equally, so that a health positioning map that is highly accurate and readily understandable can be created. In particular, both data obtained from a male subject and data obtained from a female subject can be evaluated on the same health positioning map by performing standardization processing on a parameter arising from a difference between males and females.

More preferably, processing by the processing **122** comprises weighting processing on a first data set, standardization processing on the weighted data set, and dimensionality reduction processing on the standardized data set. This is because this enables the magnitude of effect of each parameter of the first data set on a health positioning map to be emphasized and creates a health positioning map that is even more accurate and readily understandable.

Standardization processing by the processing means 122 can be configured to be performed, for example, on all parameters of a first parameter set, or on a specific parameter. Weighting processing by the processing means 122 can be configured to be performed, for example, on a first data set of all of the plurality of subjects, or on a first data set of a specific population of the plurality of subjects.

At step **S503,** the mapping means **123** of the processing unit **120** maps first data obtained at step **S502** for each of the plurality of subjects. The mapping means **123** maps n-dimensional first data onto an n-dimensional space. The mapping means **123** can output a map mapping first data of each of the plurality of subjects by mapping the first data for each of the plurality of subjects. If, for example, the first data is two-dimensional, the mapping means **123** can output a two-dimensional map by mapping the first data onto a position on a two-dimensional space, i.e., a plane.

At step **S504,** the clustering means **124** of the processing unit **120** clusters the first data mapped at step **S503** to identify a plurality of regions. The clustering means **124** can identify any number of regions by dividing the mapped first data into any number of clusters.

At step **S505,** the characterization means **125** of the processing unit **120** characterizes at least some of the plurality of regions identified at step **S504.** The characterization means **125** can be configured, for example, to characterize at least some of the plurality of regions based on information inputted into the computer system **100,** or to automatically characterize at least some of the plurality of regions independent of any input. For example, the characterization means **125** can characterize at least some of the plurality of regions based on a relative position on a health positioning map, or based on machine learning.

A health positioning map with at least some of the plurality of regions characterized is created by the aforementioned processing **500.** The created health positioning map can be utilized in processing **510,** processing **600,** or processing **700** described below.

Figure **5B** shows another example of processing in the computer system **100.** The processing **510** is processing for creating a health positioning map for data contained in some regions of the health positioning map created in the processing **500.** The processing **510** is executed in the processing unit **120** of the computer system **100.**

At step **S511,** the processing unit **120** receives an input selecting some of the plurality of regions on the health positioning map created by the processing **500.** An input selecting some of the plurality of regions is inputted, for example, via the interface unit **110** from the outside of the computer system **100.**

At step **S512,** the acquisition means **121** of the processing unit **120** acquires first data mapped to the selected region. The acquired first data can be referred to as sub-first data.

At step **S513,** the clustering means **124** of the processing unit **120** clusters the sub-first data acquired at step **S512** to identify a plurality of regions. The processing at step **S513** can be the same as the processing at step **S504.**

At step **S514,** the characterization means **125** of the processing unit **120** characterizes at least some of the plurality of regions identified at step **S513.** The processing at step **S514** can be the same as the processing at step **S505.**

A health positioning map is created for some of the plurality of subjects by the processing **510** described above. A health positioning map for some of the plurality of subjects can be a health positioning map focused on a specific population in the plurality of subjects, such as a male subject population, a female subject population, a young population (population of subjects under 40 years old), a middle age population (population of subject who are 40 or older and younger than 60 years old), or a senior population (population of subjects who are 60 or older). A plurality of regions on a health positioning map focused on a specific population can have characterization that is different from a plurality of regions on a health positioning map of all of the plurality of subjects, and can be utilized for analyzing a health status from a different viewpoint.

Figure **6** shows another example of processing in the computer system **100.** The processing **600** is processing for creating a health function. The processing **600** is executed in the processing unit **130** of the computer system **100.**

At step **S601,** the processing unit **130** prepares a health positioning map. For example, the processing unit **130** can prepare a health positioning map by acquiring a health positioning map with the first acquisition means of the processing unit **130.** A health positioning map is created based on a first data set with respect to a first parameter set described above for a plurality of subjects. A health positioning map that is prepared can be a health positioning map created by the processing **500** or processing **510,** or a health positioning map created in another manner, as long as the map is created using the first parameter set.

At step **S602,** the second acquisition means **132** of the processing unit **130** acquires a second data set with respect to a second parameter set for at least some of the plurality of subjects. The second parameter set is a part of the first parameter set. For example, the second acquisition means **132** can acquire data for some of the plurality of subjects stored in the database unit **200** via the interface unit **110.**

At step **S603,** the derivation means **133** of the processing unit **130** derives a health function that correlates the second data set of at least some of the plurality of subjects acquired at step **S602** with a position on a health positioning map of at least some of the plurality of subjects. For example, the derivation means **133** can derive a health function by machine learning. A health function can be derived, for example, for each axis of an n-dimensional health positioning map.

A health function can be, for example, a regression model or a neural network model. If a health function is a regression model, the derivation means **133** can derive each coefficient of the regression model by machine learning using a second data set as an independent variable and a coordinate on a health positioning map of a subject as a dependent variable for each of at least some of the plurality of subjects. If a health function is a neural network model, the derivation means **133** can derive a weighting coefficient of each node by machine learning using a second data set as input supervisor data and a position on a health positioning map of a subject as output supervisor data for each of at least some of the plurality of subjects.

A health function for mapping a health level of a subject on a health positioning map is created by the aforementioned processing **600.** The created health function can be utilized in the processing **700** described below. By not including a result of an invasive test in a second parameter set when creating a health function, the resulting health function would be able to identify a position on a health positioning map from data for a parameter set that does not comprise a result of an invasive test and estimate a health level of a user. Such estimation of a health level of a user from a parameter set that does not comprise a result of an invasive test enables the user to find the user's own health level regardless of the testing site. A user would be able to find the user's own health level from a simple test performed, for example, at a company, drug store, community center, cafe, home, and the like in addition to hospitals.

Figure **7A** shows another example of processing in the computer system **100.** The processing **700** is processing for estimating a health level of a user. The processing **700** is executed in the processing unit **140** of the computer system **100.**

At step **S701,** the processing unit **140** prepares a health function. For example, the processing unit **140** can prepare a health function by acquiring a health function with the third acquisition means **141** of the processing unit **140.** A health function is a function that correlates a data set with respect to a second parameter set with a position on a health positioning map. The acquired health function can be a health function created by the processing **600** or a health function created in another manner, as long as the function can correlate a user data set with a position on a health positioning map. The health positioning map can be a health positioning map created by the processing **500** or the processing **510** or a health positioning map created in another manner, as long as the map is created using a first parameter set.

At step **S702,** the fourth acquisition means **142** of the processing unit **140** acquires a first user data set with respect to a second parameter set of a user. The fourth acquisition means **142** can acquire the first user data set stored in the database unit **200,** for example, via the interface unit **110.** Alternatively, the fourth acquisition means **142** can acquire the first user data set, for example, via the interface unit **110** from a terminal apparatus of a user.

At step **S703,** the output generation means **143** of the processing unit **140** obtains a first output by inputting a first user data set into a health function. If, for example, a health function is a regression model, a coordinate on a health positioning map is outputted as a first output by inputting a first user data set into an independent variable of the regression model. If, for example, a health function is a neural network model, a coordinate on a health positioning map is outputted as a first output by inputting a first user data set into an input layer of the neural network model.

At step **S704,** the output mapping means **144** of the processing unit **140** maps a first output onto a health positioning map. Since the output obtained at step **S703** is a coordinate on a health positioning map, the output mapping means **144** can map the coordinate onto an n-dimensional space of the health positioning map.

The health status of a user can be estimated from characterization of a region to which a health level of the user is mapped by mapping the health level of the user onto a health positioning map by the aforementioned processing **700.** For example, by using a health function that does not comprise a result of an invasive test in a second parameter set, a position on a health positioning map can be identified from data for a parameter set that does not comprise a result of an invasive test to estimate a health level of a user. Such estimation of a health level of a user from a parameter set that does not comprise a result of an invasive test enables the user to find the user's own health level regardless of the testing site. A user would be able to find the user's own health level from a simple test performed, for example, at a company, drug store, community center, cafe, home, and the like in addition to hospitals.

Figure **7B** shows an example of processing following the processing **700** shown in Figure **7A****.** The processing shown in Figure **7B** is processing for estimating a health level of a user after a predetermined period has elapsed.

At step **S705,** the fourth acquisition means **142** of the processing unit **140** acquires a second user data set with respect to a second parameter set of a user. At step **S705,** this is performed after at least a predetermined period has elapsed from step **S702.** The fourth acquisition means **142** can acquire a second user data set stored in the database unit **200,** for example, via the interface unit **110.** Alternatively, the fourth acquisition means **142** can acquire a second user data set, for example, via the interface unit **110** from a terminal apparatus of a user.

At step **S706,** the output generation means **143** of the processing unit **140** obtains a second output by inputting a second user data set into a health function. If, for example, a health function is a regression model, a coordinate on a health positioning map is outputted as a second output by inputting a second user data set into an independent variable of the regression model. If, for example, a health function is a neural network model, a coordinate on a health positioning map is outputted as a second output by inputting a second user data set into an input layer of the neural network model.

At step **S707,** the output mapping means **144** of the processing unit **140** maps a second output onto a health positioning map. Since the output obtained at step **S706** is a coordinate on a health positioning map, the output mapping means **144** can map a coordinate onto an n-dimensional space of the health positioning map.

Through steps **S705** to **S707,** a health status of a user after a predetermined period can be estimated. For example, a chronological change in the health status can be identified by comparing the health status estimated at steps **S701** to **S704** with the health status estimated at steps **S705** to **S707.**

Furthermore, a future health status can be predicted based on the direction of a chronological change on a health positioning map. For example, if a health level belongs to a region characterized as healthy on a health positioning map as a result of mapping a first output and a health level belongs to a region characterized as healthy but has approached a region characterized as a life style disease risk group as a resulting of mapping a second output after a predetermined period has elapsed, the health status can be predicted as heading toward a direction of a life style disease risk.

In one embodiment, the processing **700** can be utilized to evaluate an item for improving a health status. For example, a chronological change on a health positioning map can be identified by asking a user to use the item for improving a health status for a predetermined period and comparing a first output before the predetermined period with a second output after the predetermined period has elapsed. A chronological change on a health positioning map due to use of an item for improving a health status reflects the effect of the item for improving a health status. The quality of the effect of the item for improving a health status can be evaluated by comparing with a chronological change on a health positioning map when the item for improving a health status was not used.

Furthermore, an item for improving a health status can be recommended to a user based on the direction of a chronological change on a health positioning map due to use of the item for improving a health status. For example, an item for improving a health status is generally effective for a user with a chronological change on a health positioning map in the opposite direction from the direction of a chronological change on a health positioning map due to use of the item for improving a health status. Therefore, an item having a chronological change in the opposite direction from the chronological change on a health positioning map of a user identified by the processing of steps **S701** to **S707** can be recommended to the user.

Although the examples described above while referring to Figures **5A****,** **5B****,** **6****,** **7A****,** and **7B** describe processing performed in a specific order, the order of each processing is not limited to the described order. The processing can be performed in any logically possible order.

Although the processing at each step shown in Figures **5A****,** **5B****,** **6****,** **7A****,** and **7B** was described to be materialized by the processing unit **120,** processing unit **130,** or processing unit 140 and a program stored in the memory unit **150** in the examples described above while referring to Figures **5A****,** **5B****,** **6****,** **7A****,** and **7B****,** the present invention is not limited thereto. At least one of the processing of each step shown in Figures **5A****,** **5B****,** **6****,** **7A****,** and **7B** can be materialized by a hardware configuration such as a control circuit.

The present invention is not limited to each of the embodiments described above. Various modifications can be made within the scope specified in the claims. Embodiments obtained from appropriately combining each technical means disclosed in different embodiments are also encompassed by the technical scope of the invention.

### [Examples]

### (Example 1. Creation of health positioning map)

In this Example, initial data for 232 items related to health was first acquired for 720 subjects. The initial data was acquired at Riken Kobe Campus IIB. The 232 items included both an invasive test and a noninvasive test. The values of the acquired initial data were then corrected so that the mean value was 0 and the standard deviation was 1. A portion of the data was then extracted using a method of extracting, when a plurality of pieces of data with a correlation therebetween greater than a predetermined value (specifically, a correlation coefficient of 0.9) are available, only one piece of the plurality of pieces of data among the corrected data. This portion of data included data for four basic parameters. In this Example, when there are a plurality of pieces of data with a correlation coefficient greater than 0.9, one piece of the plurality of pieces of data was extracted, but the present invention is not limited thereto. The value of the correlation coefficient can be appropriately changed. A health function capable of computing a health level of a subject more comprehensively can be created by using a correlation coefficient of 0.9 or greater.

As a result of the extraction, 81 items were extracted. The 81 items fall under the "first parameter set" in the invention. The 81 items included four basic parameters, fundamental parameter, cognitive function parameter, subjective parameter, hematological parameter, blood vessel and skin parameter, and living condition parameter.

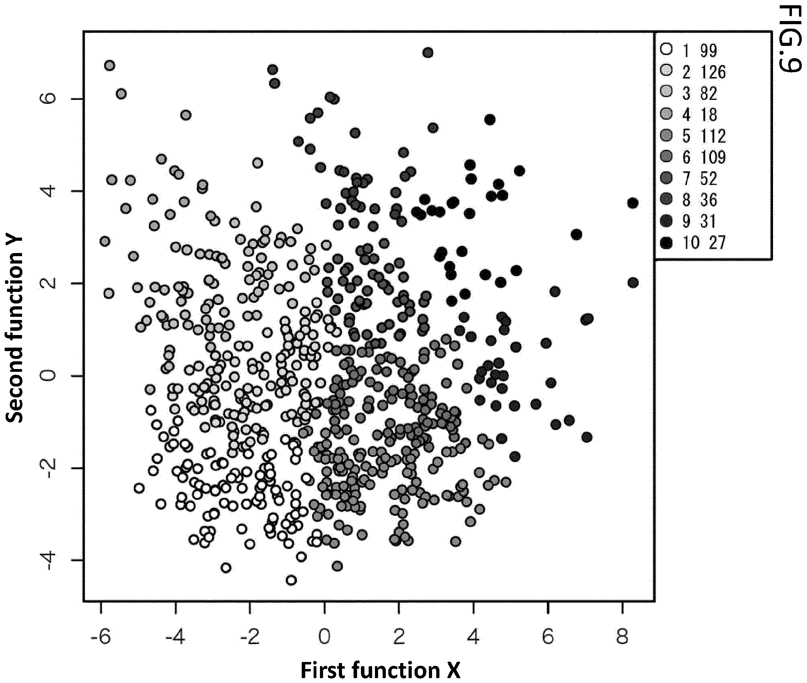

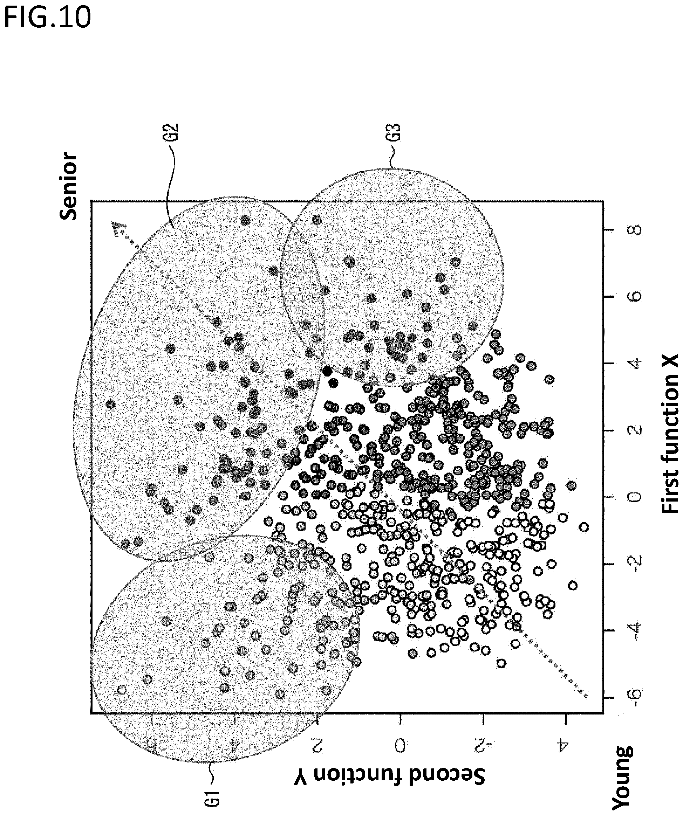

Next, multidimensional data (i.e., 81 dimensional data) was reduced to two dimensions by multidimensional scaling, and data for 692 subjects whose data were complete among the 720 members was plotted on a two-dimensional plane. A plot distribution pattern of 692 plots plotted on the two-dimensional plane was clustered by k-means into 10 clusters (Figure **9**). Each cluster was characterized with information related to health to create the health positioning map of the invention. Figure **10** is a diagram showing a health positioning map created in this Example. It should be noted that while Figure **10** also displays the actual plots, the health positioning map of the invention does not need to contain the original plots, and it is sufficient to include a region identified by clustering of plots and information related to health associated with the region.

### (Example 2. Creation of health function)

A function (health function) capable of suitable arrangement on the health positioning map created in Example 1 with fewer number of test items (second parameter set) than the first parameter set was identified by machine learning.

As described above, Figure **10** is a health positioning map in this Example. When subjects corresponding to each plot were analyzed, plots on the left side (i.e., -X axis side) in the diagram of Figure **10** were plots of relatively younger subjects, and plots on the right side (i.e., +X axis side) were plots of relatively older subjects, as shown in Figure **10****.** The subject group contained in group G1 of Figure **10** was a subject group that was young with a high degree of depression or anxiety, low autonomic nerve regulation capacity, and high number of errors in cognitive problems. Therefore, it can be understood that if a subject who has been newly measured for data belongs to group G1, the subject is likely to have a pre-disease state of a mental health disease.

A subject group contained in group G2 was a subject group that was old with high blood γ-GTP/blood ALT/blood neutral fat/blood HbA1c/blood high sensitivity CRP values. Therefore, it can be understood that if a subject who has been newly measured for data belongs to group G2, the subject is likely to have a pre-disease state of a life style disease.

A subject group contained in group G3 was a subject group that was old with a high blood sugar level. Therefore, it can be understood that if a subject who has been newly measured for data belongs to group G3, the subject is likely to have a pre-disease state of diabetes.

The regions of first function X ≤ about 4 and second function Y ≤ about 2 generally indicate a healthy group without any problems in the health status.

The above groups G1 to G3 are several examples that can be evaluated with the health evaluation apparatus of the invention. Various other health risks can also be additionally evaluated.

### (Example 3. Examination of second parameter set)

A function capable of suitable arrangement on a health positioning map with another second parameter set was attempted to be identified by machine learning.

A health positioning map was created using a first parameter set of 76 items from data for 965 subjects by adding more subjects to Example 1. In the same manner as Example 2, data for half of the subjects were used for supervisor data, and data for the remaining half of subjects was used for evaluation of the obtained function.

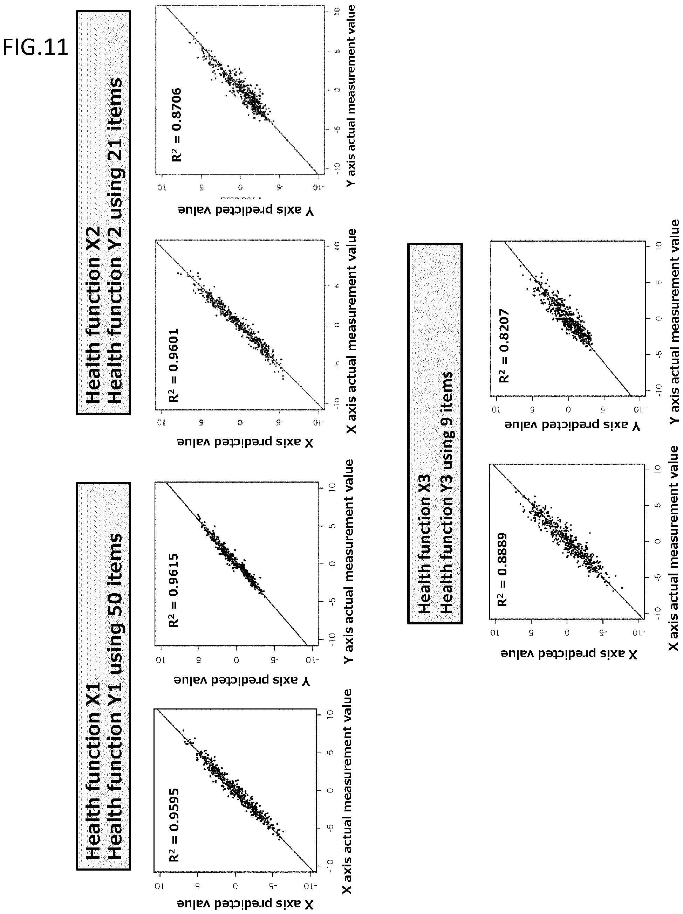

As a result, a health function from a second parameter set of 50 items (R² of X axis = 0.9595; R² of Y axis = 0.9615), health function from a second parameter set of 21 items (R² of X axis = 0.9601; R² of Y axis = 0.8706), and health function from a second parameter set of 9 items (R² of X axis = 0.8889; R² of Y axis = 0.8207) were identified (Figure **11**). In particular, it was unexpected that a health status can be evaluated with such a lower number of parameters, i.e., 9 items.

The 9 items were age, fatigue questionnaire score, fatigue duration, ln(LF+HF), cognitive function, fat percentage, blood neutral fat, OSI, and CRP.

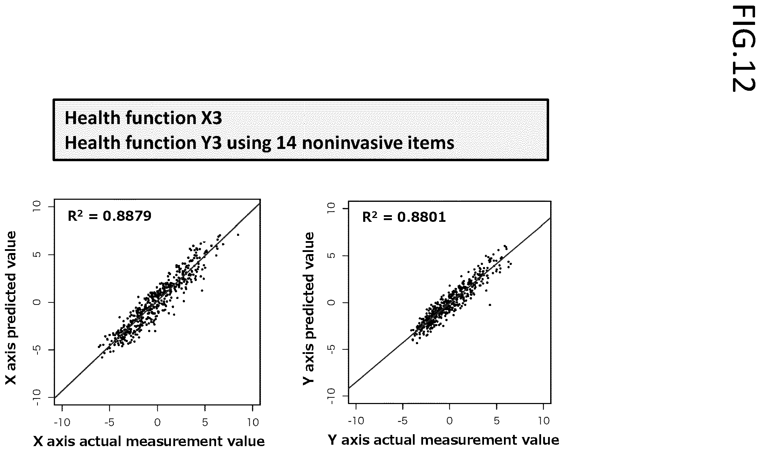

If health can be evaluated with only data from a noninvasive test, users can conveniently find their own health level regardless of the testing site. In this regard, an attempt was made to identify a second parameter set comprised of only noninvasive parameters. As a result, parameters for the following 14 items were identified, and a health function using such a parameter set was identified (Figure **12**) (R² of X axis = 0.8879; R² of Y axis = 0.8801).
*age
*BMI
*fat percentage
*SOS
*systolic blood pressure
*subjective evaluation on depression
*presenteeism
*subjective evaluation on fatigue
*activity of parasympathetic nerve (ln(HF))
*activity of an entire autonomic nervous system (ln(TP)), and
*cognitive problem.

Therefore, users can conveniently find their own health level by using the health positioning map of the invention and the 14 noninvasive parameters described above.

### (Example 4. Observation of change in health status using health positioning map)

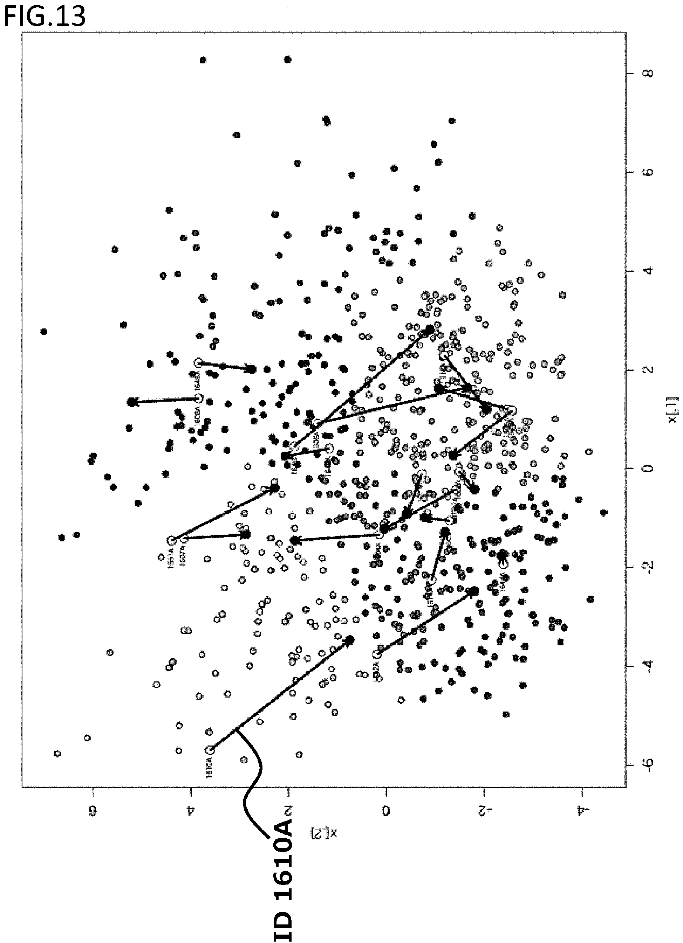

This Example studied whether a change in the health status of a subject can be observed using the health positioning map created in Example 1. Mapping positions on the health positioning map were compared between before dosing of reduced CoQ₁₀ and after dosing for 3 months. The results are shown in Figure **13****.**

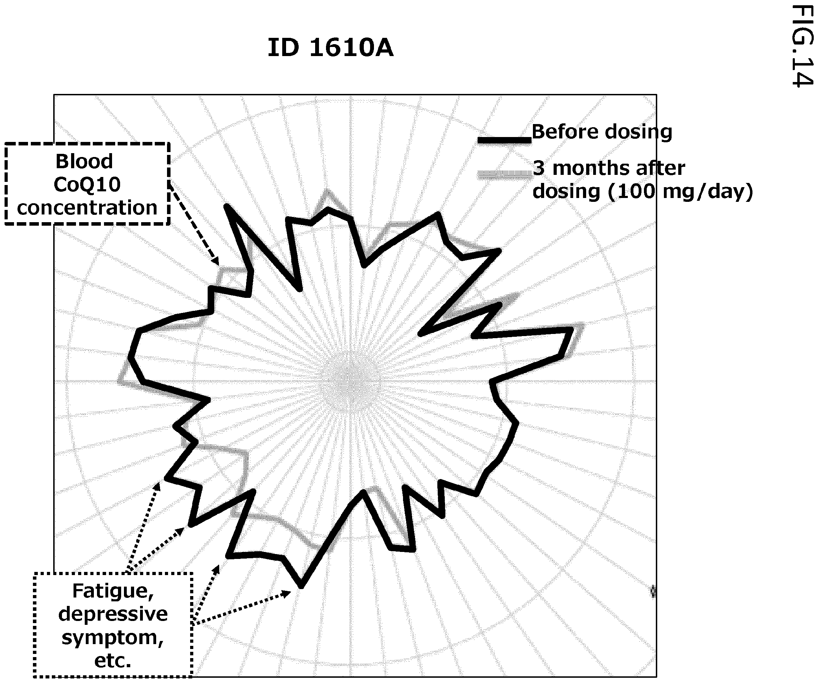

As is apparent from Figure **13****,** a change from group G1, which is a mental health risk group, to a healthy group was able to be observed for the compared subject. It was found from close inspection of each test item of the subject that the total blood CoQ₁₀ content increased, while parameters associated with fatigue or depression, i.e., fatigue VAS, depression VAS, CHATF11G, and PS, decreased (Figure **14**) . The results suggest that reduced CoQ₁₀ can be effective in eliminating fatigue or improving the mental health status and confirm that the health status of a subject can be tracked with a health positioning map and health evaluation method using the same of the invention.

### [Reference Signs List]

- **100**: Computer system
- **110**: Interface unit
- **120, 130, 140**: Processing unit
- **150**: Memory unit
- **200**: Database unit
- **300**: User terminal apparatus
- **400**: Network

## Claims

1. A method of creating a health positioning map, comprising:
acquiring a first data set with respect to a first parameter set for each of a plurality of subjects;
processing the first data set to obtain first data;
mapping the processed first data for each of the plurality of subjects;
clustering the mapped first data to identify a plurality of regions; and
characterizing at least some of the plurality of regions.

2. The method of claim 1, wherein the first parameter set comprises an autonomic nerve parameter, a biological oxidation parameter, a less biological repair energy parameter, and an inflammation parameter.

3. The method of claim 2, wherein the first parameter set further comprises a fundamental parameter, a cognitive function parameter, and a subjective parameter.

4. The method of any one of claims 1 to 3, wherein the processing of the first data set comprises dimensionality reduction processing of the first data set.

5. The method of any one of claims 1 to 4, wherein the processing of the first data set comprises standardization of the first data set and dimensionality reduction processing of the standardized data set.

6. The method of claim 5, wherein the standardization of the first data set comprises:
classifying the first data set into a data set for a male subject and a data set for a female subject; and
standardizing the dataset for a male subject and/or standardizing the data set for a female subject.

7. The method of any one of claims 4 to 6, wherein the dimensionality reduction processing is performed by multidimensional scaling.

8. The method of any one of claims 1 to 7, further comprising:
selecting the regions;
designating the first data mapped to the regions as sub-first data;
clustering the sub-first data to identify a plurality of regions; and
characterizing at least some of the plurality of regions.

9. The method of any one of claims 1 to 8, wherein the health positioning map is a two-dimensional or three-dimensional map.

10. A method of creating a health function for mapping a health level of a subject onto a health positioning map, comprising:
preparing a health positioning map created by the method of any one of claims 1 to 9;
acquiring a second data set with respect to a second parameter set for at least some of the plurality of subjects, the second parameter set being a part of the first parameter set; and
deriving a health function that correlates the second data set with a position on the health positioning map of the at least some of the subjects.

11. The method of claim 10, wherein the derivation is performed by machine learning.

12. The method of claim 10 or 11, wherein the second parameter set does not comprise a result of an invasive test.

13. The method of claim 12, wherein the second parameter set comprises:
(1) age;
(2) BMI;
(3) fat percentage;
(4) SOS;
(5) systolic blood pressure;
(6) subjective evaluation on fatigue;
(7) subjective evaluation on depression;
(8) activity of a parasympathetic nerve;
(9) activity of an entire autonomic nervous system; and
(10) cognitive function.

14. The method of claim 10 or 11, wherein the second parameter set comprises age, subjective evaluation on fatigue, fatigue duration, balance between a sympathetic nerve and a parasympathetic nerve, cognitive function, fat percentage, blood neutral fat, blood oxidative stress index (OSI), and blood CRP.

15. The method of any one of claims 10 to 14, wherein the health function is a linear regression model or a nonlinear regression model using the second data set as an independent variable and a position on the health positioning map of the at least some of the subjects as a dependent variable.

16. A method of estimating a health level of a user, comprising:
preparing a health function created by the method of any one of claims 10 to 15;
acquiring a first user data set with respect to the second parameter set of the user;
obtaining a first output by inputting the first user data set into the health function; and
mapping the first output onto the health positioning map.

17. The method of claim 16, comprising:
acquiring a second user data set with respect to the second parameter set of the user after a predetermined period has elapsed;
obtaining a second output by inputting the second user data set into the health function; and
mapping the second output onto the health positioning map.

18. A method of evaluating an item for improving a health status, comprising:
comparing the first output with the second output in the method of claim 17;
wherein the user uses the item during the predetermined period.

19. A method of creating a health function for mapping a health level of a subject on a health positioning map, comprising:
preparing a health positioning map created by using a first parameter set;
acquiring a second data set with respect to a second parameter set, the second parameter set being a part of the first parameter set; and
deriving a health function that correlates the second data set with a position on the health positioning map of a subject.

20. The method of claim 19, wherein the first parameter set comprises an autonomic nerve parameter, a biological oxidation parameter, a less biological repair energy parameter, and an inflammation parameter.

21. The method of claim 19 or 20, wherein the derivation is performed by machine learning.

22. The method of any one of claims 19 to 21, wherein the second parameter set does not comprise a result of an invasive test.

23. The method of claim 22, wherein the second parameter set comprises:
(1) age;
(2) BMI;
(3) fat percentage;
(4) SOS;
(5) systolic blood pressure;
(6) subjective evaluation on fatigue;
(7) subjective evaluation on depression;
(8) activity of a parasympathetic nerve;
(9) activity of an entire autonomic nervous system; and
(10) cognitive function.

24. The method of any one of claims 19 to 21, wherein the second parameter set comprises age, subjective evaluation on fatigue, fatigue duration, balance between a sympathetic nerve and a parasympathetic nerve, cognitive function, fat percentage, blood neutral fat, blood oxidative stress index (OSI), and blood CRP.

25. A method of estimating a health level of a user, comprising:
acquiring a first user data set with respect to a second parameter set of the user;
obtaining a first output by inputting the first user data set into a health function; and
mapping the first output onto a health positioning map;
wherein the health function is a function that correlates the user data set with a position on the health positioning map.

26. The method of claim 25, wherein the second parameter set does not comprise a result of an invasive test.

27. The method of claim 26, wherein the second parameter set comprises:
(1) age;
(2) BMI;
(3) fat percentage;
(4) SOS;
(5) systolic blood pressure;
(6) subjective evaluation on fatigue;
(7) subjective evaluation on depression;
(8) activity of a parasympathetic nerve;
(9) activity of an entire autonomic nervous system; and
(10) cognitive function.

28. The method of claim 25, wherein the second parameter set comprises age, subjective evaluation on fatigue, fatigue duration, balance between a sympathetic nerve and a parasympathetic nerve, cognitive function, fat percentage, blood neutral fat, blood oxidative stress index (OSI), and blood CRP.

29. The method of any one of claims 25 to 28, wherein the health positioning map is created using a first parameter set, and the first parameter set comprises an autonomic nerve parameter, a biological oxidation parameter, a less biological repair energy parameter, and an inflammation parameter.

30. The method of claim 29, wherein the first parameter set further comprises a fundamental parameter, a cognitive function parameter, and a subjective parameter.

31. A system for creating a health positioning map, comprising:
acquisition means for acquiring a first data set with respect to a first parameter set for each of a plurality of subjects;
processing means for processing the first data set to obtain first data;
mapping means for mapping the processed first data for each of the plurality of subjects;
clustering means for clustering the mapped first data to identify a plurality of regions; and
characterization means for characterizing at least some of the plurality of regions.

32. A system for creating a health function for mapping a health level of a subject on a health positioning map, comprising:
first acquisition means for acquiring a health positioning map created by the system of claim 31;
second acquisition means for acquiring a second data set with respect to a second parameter set for at least some of the plurality of subjects, the second parameter set being a part of the first parameter set; and
derivation means for deriving a health function that correlates the second data set with a position on the health positioning map of the at least some of the subjects.

33. A system for estimating a health level of a user, comprising:
third acquisition means for acquiring a health function created by the system of claim 32;
fourth acquisition means for acquiring a first user data set with respect to the second parameter set of the user;
output generation means for generating a first output by inputting the first user data set into the health function; and
output mapping means for mapping the first output onto the health positioning map.

34. A system for creating a health function for mapping a health level of a subject onto a health positioning map, comprising:
first acquisition means for acquiring a health positioning map created by using a first parameter set;
second acquisition means for acquiring a second data set with respect to a second parameter set, the second parameter set being a part of the first parameter set; and
derivation means for deriving a health function that correlates the second data set with a position on the health positioning map of a subject.

35. A system for estimating a health level of a user, comprising:
acquisition means for acquiring a first user data set with respect to a second parameter set of the user;
output generation means for generating a first output by inputting the first user data set into a health function; and
output mapping means for mapping the first output onto a health positioning map;
wherein the health function is a function that correlates the user data set with a position on the health positioning map.

36. A program for creating a health positioning map, the program being executed in a computer system comprising a processing unit, the program causing the processing unit to perform processing comprising:
acquiring a first data set with respect to a first parameter set for each of a plurality of subjects;
processing the first data set to obtain first data;
mapping the processed first data for each of the plurality of subjects;
clustering the mapped first data to identify a plurality of regions; and
characterizing at least some of the plurality of regions.

37. A program for creating a health function for mapping a health level of a subject on a health positioning map, the program being executed in a computer system comprising a processing unit, the program causing the processing unit to perform processing comprising:
acquiring a health positioning map created by executing the program of claim 36;
acquiring a second data set with respect to a second parameter set for at least some of the plurality of subjects, the second parameter set being a part of the first parameter set; and
deriving a health function that correlates the second data set with a position on the health positioning map of the at least some of the subjects.

38. A program for estimating a health level of a user, the program being executed in a computer system comprising a processing unit, the program causing the processing unit to perform processing comprising:
acquiring a health function created by executing the program of claim 37;
acquiring a first user data set with respect to the second parameter set of the user;
obtaining a first output by inputting the first user data set into the health function; and
mapping the first output onto the health positioning map.

39. A program for creating a health function for mapping a health level of a subject onto a health positioning map, the program being executed in a computer system comprising a processing unit, the program causing the processing unit to perform processing comprising:
acquiring a health positioning map created by using a first parameter set;
acquiring a second data set with respect to a second parameter set, the second parameter set being a part of the first parameter set; and
deriving a health function that correlates the second data set with a position on the health positioning map of a subject.

40. A program for estimating a health level of a user, the program being executed in a computer system comprising a processing unit, the program causing the processing unit to perform processing comprising:
acquiring a first user data set with respect to a second parameter set of the user;
obtaining a first output by inputting the first user data set into a health function; and
mapping the first output onto a health positioning map;
wherein the health function is a function that correlates the user data set with a position on the health positioning map.
